# EUROPEAN PATENT SPECIFICATION

(11) **EP 3 071 602 B1**
(45) Date of publication and mention of the grant of the patent: **23.08.2017**
(21) Application number: 14827154.7
(22) Date of filing: 21.11.2014
(51) Int. Cl.: C08B 37/08, A61K 8/00, C08L 5/08

(54) **NANOFIBERS CONTAINING PHOTOCURABLE ESTER DERIVATIVE OF HYALURONIC ACID OR ITS SALT, PHOTOCURED NANOFIBERS, METHOD OF SYNTHESIS THEREOF, PREPARATION CONTAINING PHOTOCURED NANOFIBERS AND USE THEREOF**
NANOFASERN MIT EINEM LICHTHÄRTBAREN ESTERDERIVAT AUS HYALURONSÄURE ODER SEINEM SALZ, LICHTGEHÄRTETE NANOFASERN, VERFAHREN ZUR SYNTHESE DAVON, PRÄPARAT MIT LICHTGEHÄRTETEN NANOFASERN UND VERWENDUNG DAVON
NANOFIBRES CONTENANT UN DÉRIVÉ ESTER PHOTODURCISSABLE DE L'ACIDE HYALURONIQUE OU DE SON SEL, NANOFIBRES PHOTODURCIES, LEUR PROCÉDÉ DE SYNTHÈSE, PRÉPARATION CONTENANT DES NANOFIBRES PHOTODURCIES ET SON UTILISATION

(30) Priority: 21.11.2013 CZ 20130914
(43) Date of publication of application: 28.09.2016
(73) Proprietor: Contipro a.s., 56102 Dolni Dobrouc (CZ)
(72) Inventor: HUERTA-ANGELES, Gloria, 560 02 Ceska Trebova (CZ); BOBEK, Martin, 27201 Kladno (CZ); PRIKOPOVA, Eva, 561 64 Techotin (CZ); RUZICKOVA, Jana, 14800 Praha 4 - Kunratice (CZ); MORAVCOVA, Martina, 56152 Vermerovice (CZ); BRANDEJSOVA, Martina, 561 51 Letohrad (CZ); VELEBNY, Vladimir, 564 01 Zamberk (CZ)
(74) Representative: Dvorakova, Martina
(86) International application number: PCT/CZ2014/000138
(87) International publication number: WO 2015/074632

(56) References cited:
- EP-A1- 1 369 441
- EP-A2- 2 522 337
- WO-A1-2013/159757
- WO-A1-2014/082608

## Description

### Field of the invention

The present invention relates to nanofibers containing a photo curable ester derivative of hyaluronic acid or its salt, photocured nanofibers and the methods of their synthesis and use of the preparation containing photocured nanofibers.

### Background of the invention

Hyaluronic acid (HA or Hyaluronan, Figure A) is a linear glycosaminoglycan consisting of alternating D-glucuronic acid and N-acetyl-D-glucosamine units. HA is non-immunogenic and therefore it has a great potential in medicine. HA characterized by high molecular weight, has been found to be particularly useful in a variety of clinical fields, including wound healing, ophthalmic surgery and orthopaedic surgery due to its visco-elastic properties. HA is also potentially useful in a variety of non-medical fields including cosmetic applications.

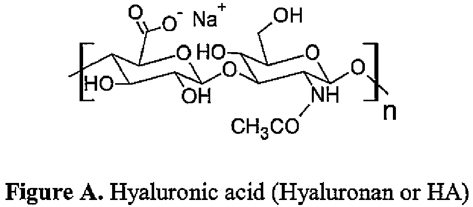

HA is a water-soluble polymer and produces highly viscous solutions. HA has been chemically cross-linked by means of different functional groups such as, epoxy compounds, divinylsulfone and the like, to prolong the half-life of the biopolymer *in vivo* as well as for preparing materials for medical use in the form of films or hydrogels. The main problem to be solved for medical and cosmetic applications of materials made thereof is the completely elimination of unreacted chemicals and/or catalysts after reaction. The elimination of these components is challenging due to macromolecular conformation of HA, however, it is very important since when it is administered or implanted into living bodies, said residual chemicals may produce adverse effects, so that they are not recommended for a practical use in biomedical field.

On previously reported art, such as US patent No. 7951936 that describes a photo-cross-linked reactive hyaluronic acid wherein the carboxylic functional groups of HA was chemically modified. The functionalities use for photo-crosslinking may include cinnamic acid. These aminoalkyl ester derivatives were bonded to the polysaccharide by an amide bond between the amino residue of the aminoalkyl group and the carboxyl group of HA. Such modification of carboxyl alter biocompatibility of the obtained materials because the carboxyl group of HA is known to be the recognition site of hyaluronidase and HA receptors.

US patent application No. 2012289478 (A1) describes ester derivatives of hyaluronic acid with hydroxy-cinnamic acid used as protective agents in dermatology. The synthesis of such esters involves the use of formamide, which is very toxic and the activation of the carboxyl group of hydroxy-cinnamic acid of derivatives thereof is done using carbonyl diimidazol (CDI). The obtained highly viscous solutions or hydrogels cannot be used for electrospinning because CDI may cause self-cross-linking of HA.

Acylation of HA has been reported in several patent documents. For example, it is catalyzed by chloroformates in WO 2010/105582. However, the application of that reagent for the functionalization of polysaccharides is still difficult due to the number of side reactions arising from their high reactivity such as described in a research article (Wondraczek, et al. Synthesis. Carbohydrate Polymers 2011, 83, (3), 1112-1118). Furthermore a conversion of hyaluronic acid to its acid form decreases the recoveries of the polymer and increases considerably the polydispersity. There is also claimed the necessity of anhydrous DMSO, which is very expensive in industrial scale. Additionally, one of the most important disadvantage is the high amount of 4-dimethyl-aminopyridine (or DMAP) used for the reaction feed. The problem of using this reagent is derived from its acute lethal effects (classified as T⁺). However, the reagent is very commonly used for acylation as acyl-transfer agent. The use of DMAP for acylation of hyaluronic acid was recently published (Šmejkalová et al. Carbohydrate Polymers, 87(2), 2012, 1460).

Examples of the O-acylation in US Patent No. 7,041,310 included reactions employing one or more acid catalysts, for example, mineral acids such as hydrochloric acid or sulfuric acid, organic acids such as aromatic sulfonic acid and Lewis acids such as boron fluoride etherate or the like, and a reaction with an organic acid employing one or more dehydrating agents, for example, N,N'-dicyclohexylcarbodiimide, 2-chloro-1-methyl pyridinium iodide and N,N'-carbonyl diimidazole or the like. However, the composition involves a complex of hyaluronic acid or a salt thereof and a quaternary ammonium salt, which is in general consider as toxic.

Similar reaction conditions were reported on Patent document WO2005092929A1 and related to hyaluronic acid butyric esters in which the hydroxyl groups of hyaluronic acid are partially esterified with butyric residues, characterized by a degree of substitution being equal or below 0.1. These esters with low degree of substitution are obtained by means of a process carried out in the homogeneous phase under anhydrous conditions, wherein hyaluronic acid is used in the form of a quaternary nitrogen salt. However, these conditions are difficult to be followed in high scale. US Patent No. 2005/0119219A1 relates to hyaluronic acid ester derivatives and hydrogels prepared by photo-curing. The main disadvantage of the synthesis is that is starting from the tetrabutylammonium salt of hyaluronic acid in anhydrous conditions, which is an expensive process and considerably degrades the polysaccharide.

US Patent No. 8247546B2 describes derivatives of acid polysaccharides, wherein the process of preparation comprises the reaction in homogeneous phase using a protic polar solvent such as formamide and an anhydride of an alkylcarboxylic acid, in the presence of a basic catalyst. There are several disadvantages of the synthetic pathway such as the use of formamide, a carcinogenic solvent that seriously limits the upscale of the reaction as well as the use of the modified polymer in medical devices. A second limiting is the use of high temperatures during the reaction (95°C), which degrades the polysaccharide. Similar conditions were used in US Patent No. 2012/0289478 A1. Synthesis of esters of hyaluronic acid and cinnamic, caffeic or ferulic acids was described and the use of the materials for cross-linking. However, this synthesis involves again the use of formamide for dissolution of the reaction mixture. US Patent No. 5,462,976 stated clearly that the synthesis of photocurable derivatives of glycosaminoglycans requires high temperature and longer reaction time so that HA is degraded and produced unknown reaction sub-products.

In similar art, Japanese Patent number JP-19940307050 provides a cinnamic-photoreactive derivative having a spacer introduced between the reactive cinnamic group and the polymeric backbone. The disadvantage of its preparation is the long synthetic pathway as well as the use of very toxic reagents such as trifluoroacetic acid that impair an effective scale up.

Several activators of the carboxylic group have been reported in the literature such as the used ones for formation of peptide bond. **Scheme 1** shows the activation of the carboxylic group with thionyl chloride to form the acid chloride, published recently in Molecules 2012, 17, 8661-8673. However, not all of such activators can be considered to be used for the modification of hyaluronic acid in aqueous solution since they may react violently and/or explosively and release dangerous gases upon contact with water and other reagents on the reaction feed.

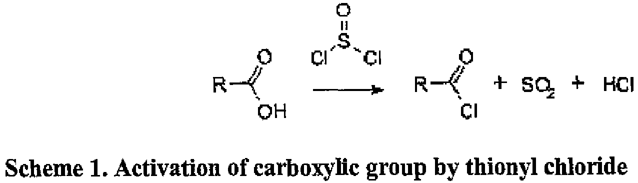

On the other hand, electrospinning is a highly versatile method that produces fibers on the micro- or nano-scale. The technique of electrospinning of liquids and/or solutions is well known and described in previous art, such as U.S. Patents No. 4,043,331 and U.S.5,522,879. The process of electrospinning involves the introduction of a liquid into an electric field, so that the fibers are produced. These fibers are generally drawn to a conductor at an attractive electrical potential for collection. During the conversion of the liquid into fibers, the fibers harden and/or dry. This hardening and/or drying may be caused by cooling of the liquid, ie., where the liquid is normally a solid at room temperature; by evaporation of a solvent, or by a curing mechanism. As an example, US Patent Application No. 2011/0020917-A1 describes the electrospinning of poly-ε-caprolactone for the immobilization of biologically active compounds. There are some research papers related to electrospinning of polymers such as poly (vinyl alcohol) (PVA), but the authors make clear that the derivatives of PVA are not reactive enough for cross-linking, thereafter, the process requires heat treatment to favor the reaction (Zeng et al, Macromolecular Rapid communications, 2005, 26, 157). However, preheating is not favorable for sensitive polymers such as HA that considerably degrades with heating.

US Patent number US20110111012 describes the formation of wound dressings that use genipin, diethyl squarate or oxalic acid as cross-linking agents. However the incorporated cross-linking agents can be difficult to eliminate from the dressings. Another disadvantage of the process is high temperatures (150 °C) during drying, which cannot obviously be applied to HA.

US Patent Application No. 2010/002155 describes the external application of wound dressings made from nanofibers. However, this kind of material cannot be used as well for internal use due to the presence of silver particles, which are not absorbable and biodegraded in the body.

There are several research articles describing the electrospinning of native hyaluronic acid. The articles mainly deal with blends consisting of HA acid/gelatin (HA/GE) obtained in *N, N*-dimethylformamide and water-mixed solvents (Li et al. Macromolecular Rapid Communications 2006;27(2):114-120). Unfortunately, the use of DMF is not preferred for materials designed for biomedical applications due to its high toxicity. US Patent No. US20080071001 relates to the process of preparation of a polysaccharide sponge, wherein the cross-linking formation involves the cryo-processing of the material by cooling the solution to -80°C.

Generally as mentioned above there are several disadvantages and problems concerning the process of preparation of material made from HA derivative applicable in biomedical field comprising the presence of toxic residues in the material that have to be removed in the complicated and expensive way, additionally using reaction conditions that are not suitable for HA, such as high temperature and anhydrous conditions.

### SUMMARY OF THE INVENTION

The problems mentioned above are solved in the present invention concerning nanofibers based on photocurable ester derivative of hyaluronic acid or its salt and method of synthetic thereof The photocurable ester derivatives of hyaluronic acid or its salt are suitable for forming of nanofibers by electrospinning and the photocured preparation from such nanofibers that is suitable for internal and external medical use. The main advantages of such method are performing the method using not extremely toxic components under the mild reaction conditions, especially using mixture of water and water-miscible polar or non-polar solvent. Furthermore, the nanofibers cam be easily photo-cured (stabilized) by light irradiation - mediated cross-linking in the absence of initiators or activators.

The subject-matter of the inventions concerns nanofibers comprising a photocurable ester derivative of hyaluronic acid or its salt of the general formula IV 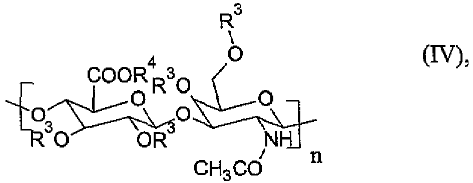
wherein n is integer in the range of 1 to 5000 dimers.
R³ are independently H or COCHCHR¹ wherein R¹ is 5- or 6-membered aryl or 5- or 6-membered heteroaryl having at least one or more identical or different heteroatoms selected from the group comprising N, O, S, preferably phenyl, furyl, furfuryl, thienyl, thiofenyl, pyridyl or imidazoyl;
provided that at least one R³ of the derivative is COCHCHR¹;
   R⁴ is H⁺ or pharmaceutically acceptable salt, preferably selected from a group comprising any of ions of alkali metals or ions of alkaline-earth metals, more preferably Na⁺, K⁺, Mg²⁺ or Li⁺;
   and at least one carrying polymer.

The HA or its salt is mainly modified at least at one position of OH groups of D-glucuronic acid or N-acetyl-glucosamine unit of HA, preferably at position C6 of the N-acetyl-glucosamine of HA and randomly to the positions C2, C3 of glucuronic acid or C4 of N-acetyl-glucosamine.

Amount of photoreactive groups (-COCHCHR¹) in the photo curable ester derivative of hyaluronic acid or its salt of the invention is from 0.1 to 20 % per 100 dimer of hyaluronic acid or its salt, preferably 5 to 10 %, more preferably 5%. Such amount of photoreactive groups corresponds the degree of substitution (DS) that was determined by integration of the signals on ¹HNMR (heteroaromatic moieties corresponding to for example furan, thiophene, pyridine, imidazole and the like) related to the methyl group corresponding to the N-acetyl group.

The carrying polymer is selected from a group comprising polyvinyl alcohol (PVA), polyacrylic acid (PAA), polyethylene oxide (PEO), polyvinyl pyrrolidone (PVP).

An amount of the carying polymer in the nanofibers of the present invention is in the range of 50% (w/w) to 99% (w/w), preferably in the range of 70 %(w/w) to 90% (w/w), more preferably 80% (w/w).
The nanofibers according to the invention can comprise biologically compatible polymer relatively easily spinnable and biologically compatible polymer preferably carboxymethyl cellulose (CMC) gelatin, chitosan, polycaprolactone (PCL), polylactic acid (PLA), polyamide (PA), polyurethane (PUR), poly-(lactide-co-glycolic) acid; and their mixture or their copolymers.

Another aspect of the present invention is a method of synthesis of nanofibers of the present invention containing the photo curable ester derivative of hyaluronic acid or its salt of the general formula IV as defined above comprising
an activation reaction of α,β-unsaturated substituted acrylic acid of the general formula I 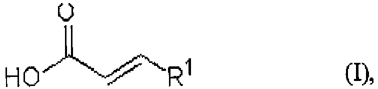 wherein
R¹ is is 5- or 6-membered aryl or 5- or 6-membered heteroaryl having at least one or more identical or different heteroatoms selected from the group comprising N, O, S;
with substituted or non-substituted benzoyl chloride or its derivatives of the general formula II 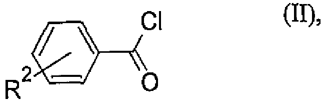 wherein R² is one or more substituents selected from a group comprising H, -NO₂, -COOH, halides, C₁-C₆alkylkoxy, preferably halides, methoxy or ethoxy, more preferably Cl; in the presence of an organic base that means aliphatic amine preferably a tertiary amine having a linear or branched, saturated or unsaturated, substituted or non-substituted C₃-C₃₀alkyl group, preferably tertiary amine, more preferably triethylamine (TEA), diisopropylethylamine (DIPEA), N,N-Diisopropylethylamine (DIEA) ; a mixture of water and water-miscible polar or non-polar solvent to form
a reactive anhydride of the general formula III 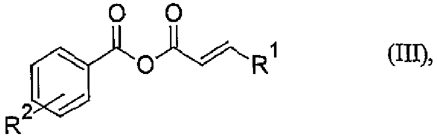 wherein
R¹ and R² are as defined above, (see also Scheme 2 below, Step A, an activation step),
that reacts with hyaluronic acid or a salt thereof in the presence of an organic base, that means aliphatic amine preferably a tertiary amine having a linear or branched, saturated or unsaturated, substituted or non-substituted C₃-C₃₀ alkyl group, preferably tertiary amine, more preferably triethylamine or diisopropylethylamine; a mixture of water and water-miscible polar or non-polar solvent, (see also Scheme 2 below, Step B, a modification step), to form the photocurable ester derivative of hyaluronic acid or its salt of the general formula IV as defined above that is dissolved in the carrying polymer of molecular weight from 5 x 14⁴ g/mol to 1 x 10⁶ g/mol, preferably 4 x 10⁵ g/mol in water to form a spinning solution that is spun by electrospinning.

The spinning solutions can be formed with PEO of different molecular weights depending of the specific application.

The concentration of the photocurable ester derivative of hyaluronic acid or its salt in the spinning solution is in the range of 0.5 to 12% (w/w), preferably 5 to 7.5% (w/w), more preferably 5%(w/w).

The substituents R² of benzoyl chloride or its derivatives of the general formula II as defined above can be located in positions *ortho-, metha-* or *para-* to the acyl chloride-group, preferably in *ortho-* or *para-* positions. The use of benzoyl chloride and its derivatives as the activators is not generally used for chemical modification of HA because it is believed that catalyzes transesterification reactions and it may react with common organic solvents used for the chemical modification of HA and respective isolation and purification, such as ethanol, methanol and higher alkyl-alcohols.

According to one preferred embodiment of the invention and also Scheme 2 below (see Part A), the activation step is carried out by mixing of 5-membered or 6-membered heteroaromatic acrylic acid of general formulae Ia or Ib below 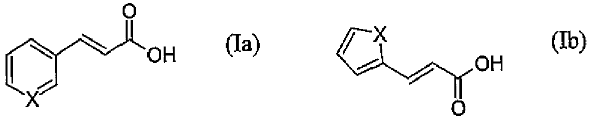 wherein X is selected from O, S, N, preferably O or N, preferably 3-(2-furyl)acrylic acid, with benzoyl chloride as the activating agent.

The activation step may be performed for 5 to 120 minutes at any temperature between 0 °C to 60°C. However, if the temperature is higher than 60°C, the reaction may generate sub-products, while if the reaction is cooled down the reaction will become slow but the reaction is still proceeding. Therefore, it is preferable to carry out the reaction at a temperature of 25 °C to 60 °C, more preferably for 30 minutes at 25°C.

The activation of the benzoyl chloride with any one of derivative of the Figures Ia or Ib is carried out almost instantly. However, the reactive intermediate of the structure III can be prepared by reacting the intermediate Ia or Ib with II under vigorous stirring for two to five hours, in the presence of a tertiary amine. The reaction time can be changed due to stirring factors, whether the reaction is carried out in higher scale.

Moreover, the reaction depicted in the Part B is carried out without the presence of 4-dimethylaminopyridine (DMAP), an organocatalysts commonly used in acylation of alcohols. This catalyst is very toxic and not acceptable for materials intended for medical use. This is a great advantage of this methodology compared to the previously reported art. Additionally, it was found that using of the combination benzoyl chloride or its derivatives of the general formula II and DMAP produces higher degree of substitution (>30%) and self crosslinking of the HA derivatives. This is undesirable since it precludes the use of the derivatives of hyaluronan for electrospinning (see Examples 14 and 16)

### A) An activation of α,β-unsaturated substituted acrylic acid and a formation of the reactive anhydride - the activation step.

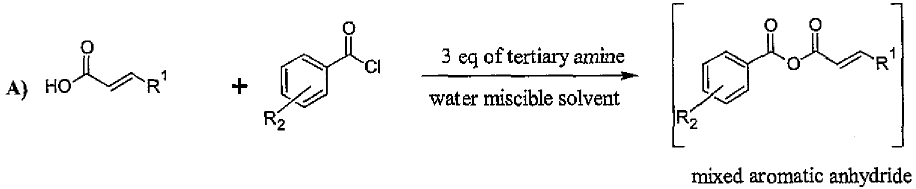

### B) A formation of the photocurable ester of HA or its salt - the modification step

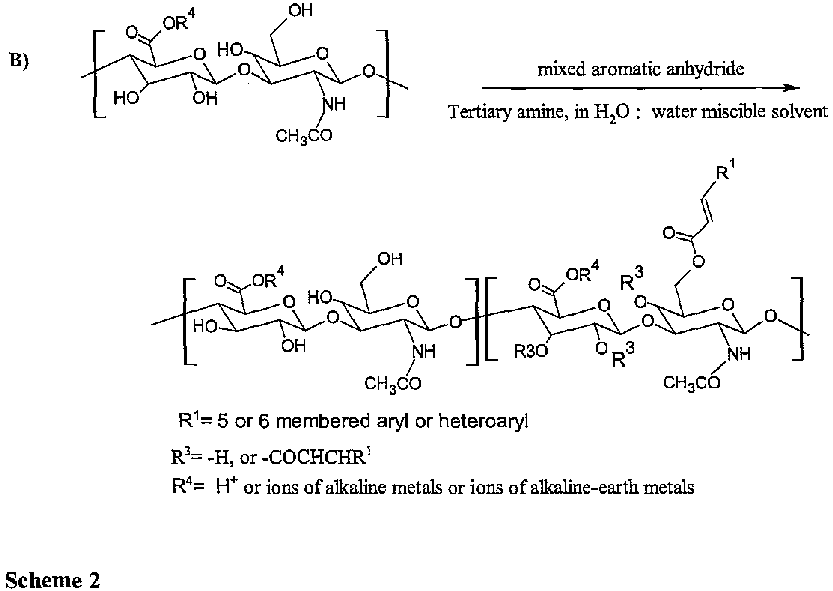

Moreover the hyaluronic acid or its salt, used in the modification step according to the method of the invention has a molecular weight (weight average of molecular weight) from 5x10³ to 1,6x10⁶ g/mol, preferably from 1.5x10⁴ to 2.5x10⁵ g/mol, more preferably 8.5x10⁴ to 1.2x10⁵ g/mol. However the method of the invention is not limited to these scopes of HA or its salt. The mean molecular weight and number average of molecular weights of the polymers were determined by SEC-MALLS as well as the polydispersity of the derivatives.

The reaction of the part B (see Scheme 2 above, the modification step) is carried out for 1 to 48 hours, preferably 1 to 3 hours; at temperatures in the range of 0 °C to 80 °C, preferably from 20 °C to 60 °C, more preferably at 25°C.

Amount of reactive anhydride of the invention mixed with HA or its salt corresponds to 0.01 to 5 equivalents, preferably 0.5 to 2 equivalents, more preferably 0,5 equivalents per dimer of hyaluronic acid or its salt to produce the photocurable ester derivative of HA or its salt of the invention having covalently bonded the photocurable group (-COCHCHR¹). The photocurable ester derivatives hyaluronic acid or its salt of the invention have not considerably changed the viscosity and physico-chemical characteristics of HA and they are characterized by low polydispersity. Moreover, the molecular weight of the derivatives has shown a slightly increased after the chemical modification. These derivatives were fully characterized by ¹H and HSQC-NMR, IR and UV spectroscopies.

The steps of the activation and the modification (see Scheme 2 above) are carried out as described above in the mixture of water and a water miscible polar or non-polar solvent miscible with water. The solvent is selected from a group comprising isopropanol, acetone, ethyl acetate, dimethyl sulfoxide, acetonitrile, dimethylformamide, tetrahydrofurane, but is preferably the use of isopropanol. The amount of water in the mixture water and water miscible polar solvent is from 10 %v/v to 99 %v/v, preferably 50 %v/v.

Additionally, this methodology of the above described method of the invention allows an easily upscale to allow the preparation of nanofibers of the invention comprising higher amounts of the photocurable ester HA-derivatives.

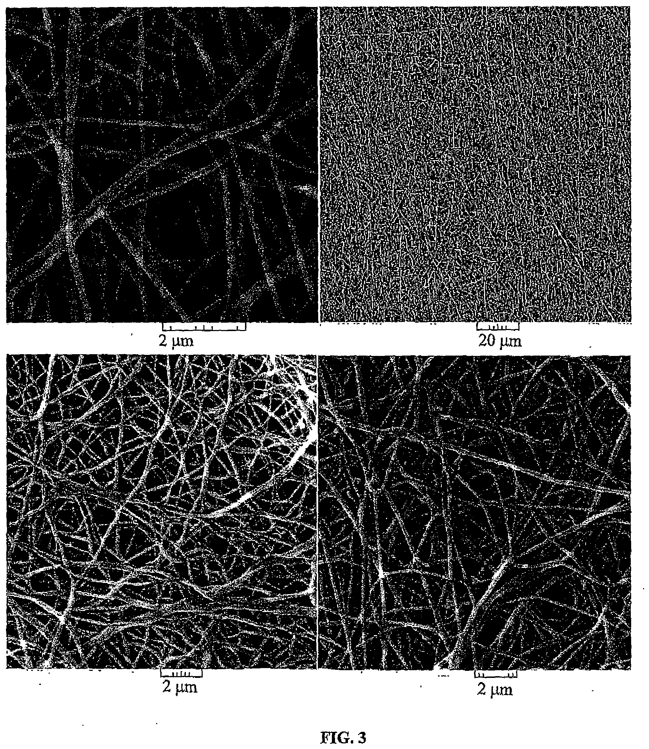

The nanofibers in accordance to the present invention can be prepared for example by electro-spinning in the device for preparation of nanofibers (for example 4SPIN^{®}, CONTIPRO-group s.r.o.) or by another electrospinning method known in the art, for example uniaxial electro spinning, coaxial electrospinning, or multiaxial electrospinning. The characterization of the fiber mesh was effected by scanning electronic microscopy (SEM) as described in the known art and shown in Figures 3, 4, 5.

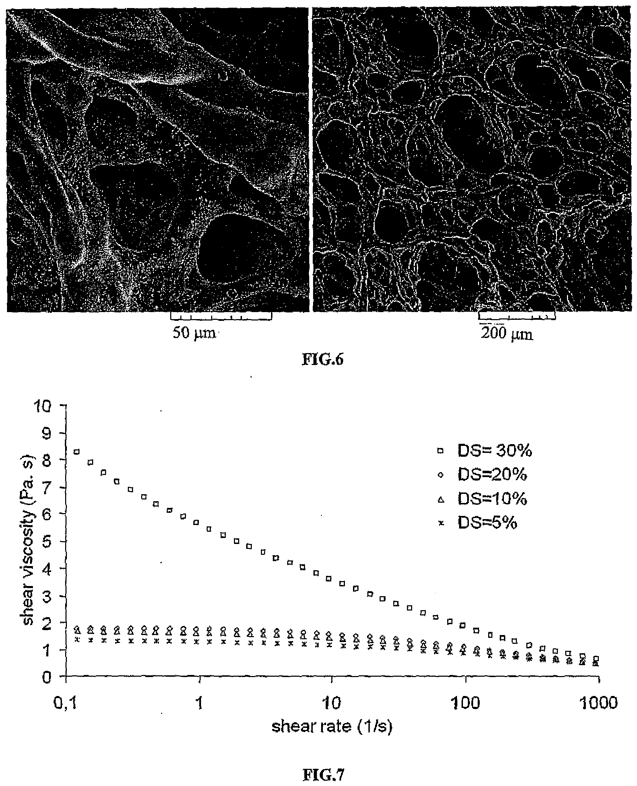

Photocurable ester derivatives of the invention possess different degree of substitution (DS). All of them were tested for the electrospinning (Table 1, Examples 22, 23 and 24). According to experimental data the ester HA-derivatives of the invention having lower degree of substitution (5-20%) are easily electrospun and also they have shown a slightly increase of viscosity due to the modification (see Figure 6). The method of chemical modification of HA as described in the modification step (see Scheme 2) is able to give the required viscosity for the electrospinning, which is very important for obtaining a large recovery (a large amount of product obtained from the process).

The nanofibers of the present invention as described above are preferably gel-forming and the average diameter thereof is between 99 to 150 nm.

The obtained fibers are able to retain their structural integrity on absorption of exudate. Alternatively the biologically compatible polymers contained in the nanofibers of the present invention increase the mechanical stability of the nanofibers as itself as well as of the final preparation.

The gel-forming fibers have preferably an absorbency in the range of at least 2 to 25 grams of 0.9% saline solution per gram of nanofiber (as measured by the free swell method), preferably at least 10 g/g. Such low water absorption capacity (about 20%) means that the materials made thereof are mechanically robust.

Additionally according to another embodiment nanofibers of the invention nanofibers as defined above can be photocured by the light irradiating within the range of UV-Vis wavelengths whereby cycloaddition of at least two ester groups of at least two molecules of hyaluronic acid or its salt of the formula IV occurs, forming the compound having cyclobutane ring of the general formula V 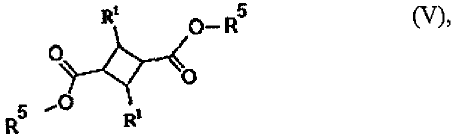 wherein
R¹ is as defined above and
R⁵ is a backbone chain of hyaluronic acid or its salt.

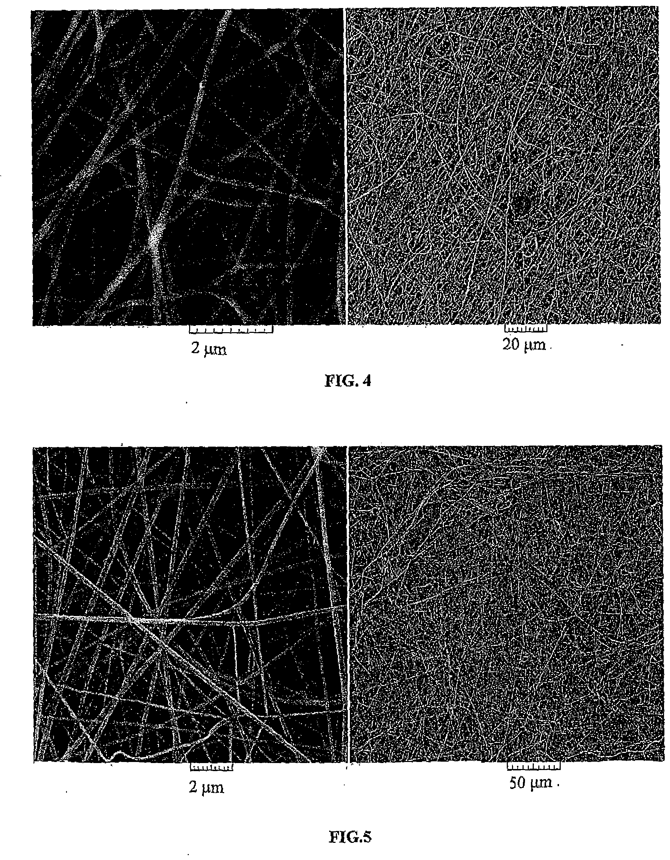

The light irradiating is done in the range of UV-Vis wavelengths, preferably in the range from 280 nm to 750 nm, more preferably at 302 nm. The irradiation according to the invention is preferably carried out at room temperature, in an exposure time between 2 minutes and 60 minutes, and more preferably between 3 to 10 minutes. After cross-linking the fibers mat is interconnected and forms a 3D network (See Figure 5).

Figure 7 shows UV-Vis spectrum of sodium 2-furyl acrylic hyaluronate nanofibers irradiated for different periods of time at 302 nm to induce cross-linking. Different aliquots were taken from the soluble part of the nanofibers after swelling in water. As it is observed the intensity of the absorption maxima decreased. With increase of UV irradiation time a significant decrease was observed. After exposure to UV-light for 60 minutes almost no absorbance was observed. These materials after electrospinning perfectively react in the solid state without the presence of initiators neither activators and are photo-cured. The photo-curing can be as well effected by a free radical polymerization process.

The photocured nanofibers of the invention comprising the compound having cyclobutane ring of the formula V, as defined above are preferably a part of the preparation which is in the form selected from a group comprising layer, film, mat, wound dressing or tissue scaffold used in cosmetics, medicine or regenerative medicine for example in wound care device on patches for external or internal use.

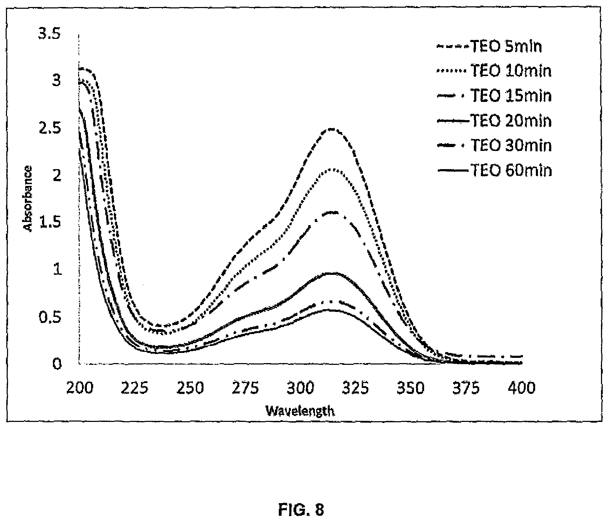

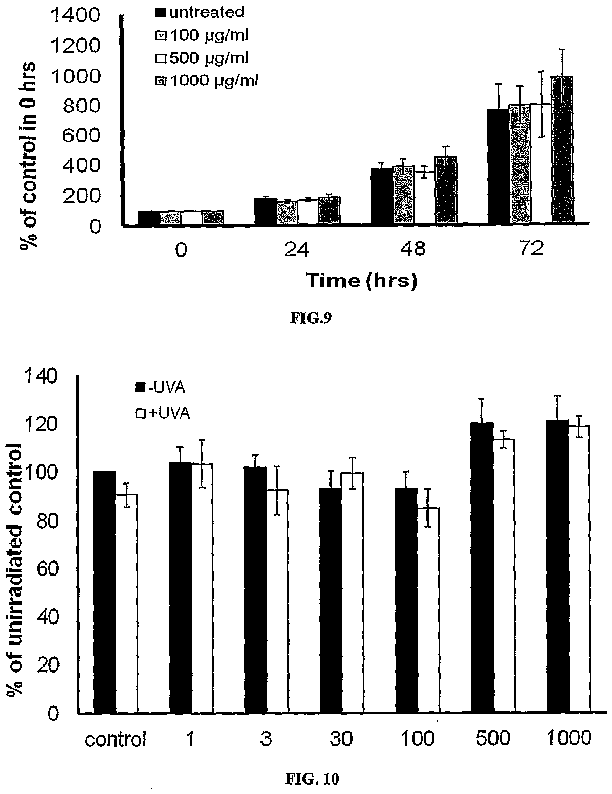

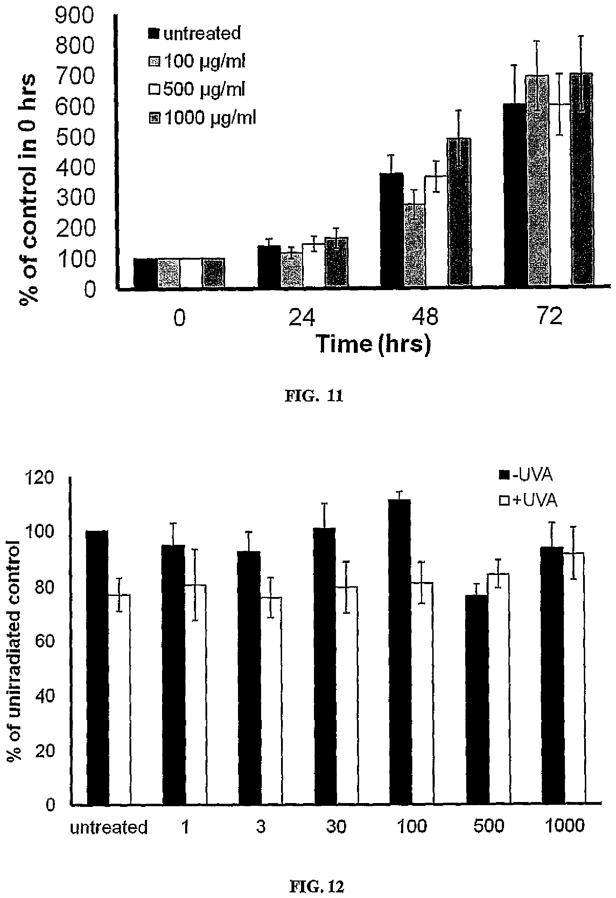

The biocompatibility of the photocurable ester derivatives of the present invention was determined by 3T3-NIH fibroblast cells cytotoxicity study (Figure 8 and 10), included as examples in this document. The biocompatibility of the products was evaluated as well as their photo-cytotoxicity (Figures 9 and 11). Thus, some examples have demonstrated that the nanofiber layer produced according to the present invention can be used to cover different wounds because the films made thereof have been in contact with fibroblasts (fibrous tissue cells) without apparent cytotoxicity. Some other cells or a combination thereof can be cultivated on the nanofiber layer. The nanofibers described in the present invention can be used for the formation of layer, film, mat, or coating that is a part of a wound dressing, or tissue scaffold form but not limited to this group.

The preparations of the present invention such as the films are not soluble in water anymore after crosslinking and can be applied to a specific point as gel or bandage made thereof The preparation of the invention can be used in cosmetics, medicine or regenerative medicine, preferably in wound care device, or in patches for the external or internal use, as it is expected for chemical modified derivatives of HA.

Advantages of the method of synthesis the nanofibers comprising photocurable ester derivate of the invention can be found in simplicity, low cost price and also that it can be carried out at mild reaction conditions using non-toxic reagents. The derivatives of the present invention were classified as biocompatible and non-cytotoxic, thus, they are suitable for in *vivo* use. They can be easily electrospun in highly efficiency. The method of photo-curing is fast and efficient and is characterized by been effected without the use of an external catalyst or initiator.

### The definitions of the terms

The term "halides" means F, Cl, Br or I.

The term "mixed aromatic anhydride" means substituted benzoic arylpropen-2-enoic anhydride or benzoic heteroarylpropen-2-enoic anhydride, or an organic component characterized by the formula 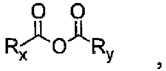 wherein Rₓ≠R_{y}, in other words a non symmetrical acid anhydride.

The term "organic base" is an aliphatic amine preferably a tertiary amine having a linear or branched, saturated or unsaturated, substituted or non-substituted C₃-C₃₀alkyl group.

The term "HA dimer" means repeating disaccharide units of hyaluronic acid. That means glucuronic acid and N-acetyl glucosamine.
The term "pharmaceutically acceptable salt" as used herein, means those salts of ester derivatives of HA of the invention that are safe and effective for topical *in vivo* use and that possess a desired biological activity. Pharmaceutically acceptable salts include salts of acidic or basic groups present in of ester derivatives of HA of the invention, preferably ions of alkali metals or ions of alkaline-earth metals, more preferably Na⁺, K⁺, Mg⁺ or Li⁺.

The term "degree of substitution of the photocurable ester HA-derivative" is the (average) number of COCHCHR¹ attached per 100 dimers of HA or it salt. For example 5 % means that 5 of each 100 dimers of HA can be substituted with a group COCHCHR¹.

The term "photo-curing" means photo-polymerization or solid state cross-linking. The term mostly refers to a process characterized by free radical or cationic photo-initiated polymerization, which can produce a cross-linking or interpenetrating polymeric network.

The term "gel-forming nanofiber" means that the fibers are hygroscopic which upon the uptake of wound exudate become moist, slippery or gelatinous and thus reduce the tendency for the surrounding fibers to adhere to the wound.

The term "film" means a complex structure composed of nanofibers characterized by a high surface area and a high porosity and is prepared by electrospinning or the like technique. These fibers are characterized by a diameter ranging from 50 nm to 1000 nm or greater. The nanofibers, which can be cross-linked or not, comprising the ester derivatives of hyaluronic acid or its salt described in this invention, can be used for coating or wrapping.

The term "carying-polymer" is referred to a polymer which is biocompatible and electrospinable. In other words, it is a natural or synthetic polymer which is intended for pharmaceutical applications and it is not causing any undesirable or systemic toxicity.

### Brief description of the drawings

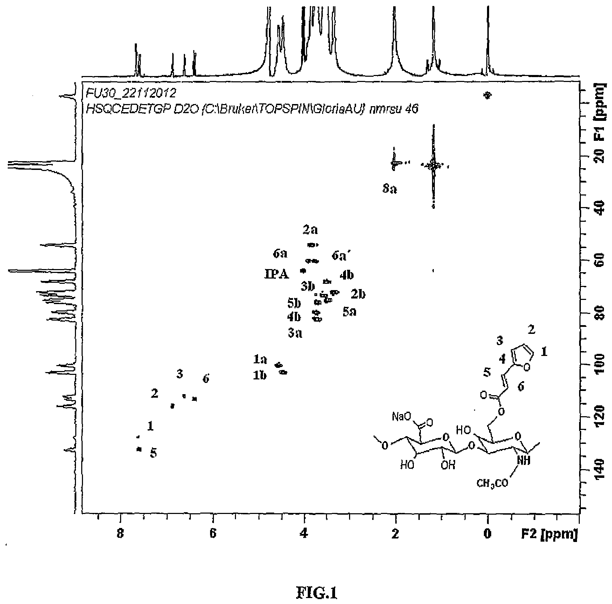

**Figure 1****.** HSQC spectra of Sodium 2-Furyl acrylate Hyaluronate obtained by the reaction described in Example 1
**Figure 2****.**¹H spectra of sodium trans-3-(3-Pyridyl) acrylic Hyaluronate
**Figure 3**. SEM micrographs displaying the morphology of fibrous mats after electro-spinning, observed at two different amplifications (A) 2 µm and (B) 20 µm (C) after 10 minutes irradiation (D) after 20 minutes irradiation (302 nm), which corresponds to Example 22, Table 1 (Example 2)
**Figure 4****.**SEM micrographs displaying the morphology of fibrous mats electro-spun which corresponds to material described in Example 22, Table 1 (Example 3), observed at A) 2 µm and (B) 20 µm.
**Figure 5****.** SEM images of fibers electrospun from samples described in Example 24
**Figure 6****.** SEM micrographs depict the morphology of fibrous mats electro-spun after cross-linking of the derivative (10 minutes) and swelling for 24 hours in PBS, as described in Example 27.
**Figure 7****.** Dynamic viscosity of the samples before spinning, showing a progressive increase of viscosity as a function of the degree of substitution
**Figure 8****.**UV-Vis spectrum of Sodium 2-Furyl acrylic Hyaluronate nanofibers irradiated for different periods at 302 nm.
**Figure 9****.** Sodium 2-Furyl acrylic Hyaluronate and its influence on cell viability tested on cells 3T3-NIH, MTT, n=6
**Figure 10****.** Sodium 2-Furyl acrylic Hyaluronate and phototoxicity assay tested on concentrations (c=1-1000ug/ml),3T3-NIH cell line, MTT, T24, n=5.
**Figure 11****.** Sodium Thiophenyl-acrylic ester of Hyaluronan and its influence on cell viability tested on cells 3T3-NIH, MTT, n=6.
**Figure 12****.** Sodium Thiophenyl-acrylic ester of Hyaluronan and phototoxicity assay tested on concentrations (c=1-1000ug/ml),3T3-NIH cell line, MTT, T24, n=5.

### Examples

### Example 1. Activation of 2-Furyl acrylic acid by benzoyl chloride and reaction with Hyaluronan

Sodium Hyaluronate (1.0 g, 2.5 mmol) with a mean molecular weight of 1.5x10⁴ g/mol was dissolved in 20 ml of distilled water. Isopropanol (IPA, 10 ml) was slowly added to that solution to avoid a possible precipitation of the polysaccharide. Subsequently, 0.7 ml of triethylamine corresponding to 2 equivalents to HA dimer was added to the reaction mixture. In a second flask, 0.17 g (1.25 mol) of 2-furyl-acrylic acid was dissolved in isopropanol (10 ml). After that 0.7 ml of triethylamine (TEA) (corresponding to 2 equivalents to HA dimer) were added to the reaction, followed by the addition of 0.15 ml of benzoyl chloride (corresponding to 0.5 equivalents of HA dimer). The activation was carried out for 30 minutes at room temperature (25°C). After that, the solution was added to the first solution (containing HA). The reaction is carried out for 3 hours at room temperature. The reaction was finished by the addition of water, and a solution of sodium chloride. After that the product was precipitated with an excess of isopropanol (250 ml). The product was washed one time by using 50 ml of isopropanol (100%) after that 4 times washed using 50 ml of mixtures of isopropanol and water (85% v/v). Finally, the precipitate was washed three times more with 100% isopropanol and dried in an oven at 40°C. Yield of the reaction 95%, Mw of the derivative (SEC-MALLS) = 1.21x10⁴ g/mol and polydispersity= 1.19.

### Chemical description of the derivative

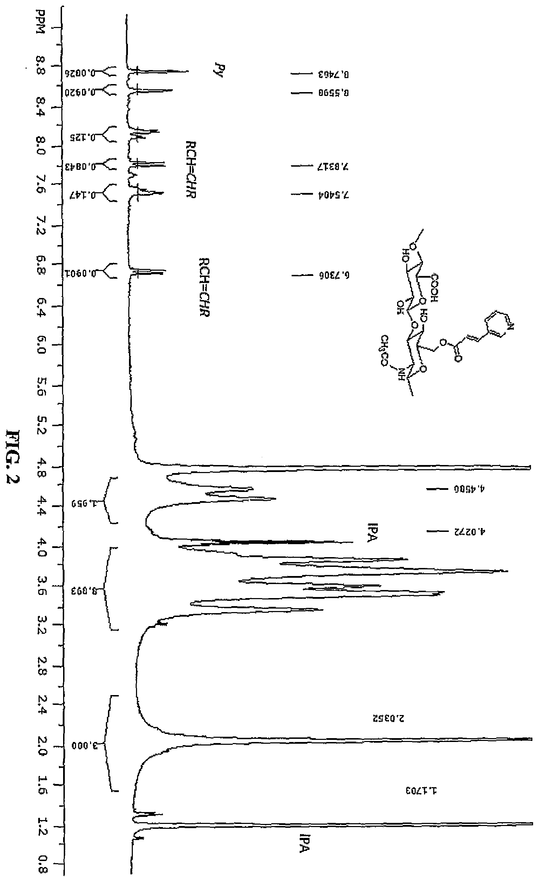

Degree of substitution was calculated to be 2.5% according to the ¹H NMR.
**¹H** NMR (500 MHz, D₂O), δ ppm, for linker: 6.40 (d, 1H), 7.18 (t, 1H), 7.47 (s, 1H), 7.62(d,1H), 7.95(d, 1H)
**2D HSQC (****Figure 2****),** δ ppm: 6.4-114.9 (signal 1), 7.18-128.6 (signal 4), 7.47-132.2 (signal 3), 7.62-129.7 (signal 5), 7.95-139.1 (signal 2).
**H-H COSY:** cross-peak signal 6.40-7.95 ppm, 7.19-7.62ppm, 7.47-7.19 ppm.
**DOSY** NMR (500 MHz, D₂O) 6.40-7.95 ppm: -11 to -12 log (m²/s), 1.90-4.70 ppm: -11 -12 log (m²/s)
UV/vis (0.05 %; H2O) λmax = 309 nm.

### Example 2. Activation of 2-Furyl acrylic acid by benzoyl chloride and reaction with Hyaluronan

Sodium Hyaluronate (1.0 g, 2.5 mmol) with a mean molecular weight of 4.0 x 10⁴ g/mol was dissolved in 20 ml of distilled water. Isopropanol (10 ml) was slowly added to that solution to avoid a possible precipitation of the polysaccharide. Subsequently, 0.7 ml of triethylamine corresponding to 2 equivalents to HA dimer was added to the reaction mixture. In a second flask, 0.17 g (1.25 mmol) of 2-furyl-acrylic acid was dissolved in isopropanol (10 ml). After that 0.7 ml of TEA (corresponding to 2 equivalents to HA dimer) were added to the reaction, followed by the addition of 0.15 ml of benzoyl chloride (corresponding to 0.5 equivalents of HA dimer). The activation was carried out for 30 minutes at room temperature (25°C). After that, the solution was added to the first solution (containing HA). The reaction is carried out for 3 hours at room temperature. The reaction was finished by the addition of water, and a solution of sodium chloride. After that the product was precipitated with an excess of isopropanol (250 ml). The product was washed one time by using 50 ml of isopropanol (100%) after that 4 times using 50 ml of mixtures of isopropanol and water (85% v/v). Finally, the precipitate was washed three times more with 100% IPA and dried in an oven at 40°C. The yield of the reaction 98.8%. %, Mw of the derivative (SEC-MALLS) = 4.2x10⁴ g/mol and polydispersity= 1.5.

### Chemical description of the derivative

Degree of substitution was calculated to be 3.5 mol % according to the ¹H NMR.
1H NMR (500 MHz, D₂O), δ ppm, for linker: 6.40 (d, 1H), 7.18 (t, 1H), 7.47 (s, 1H), 7.62(d,1H), 7.95(d, 1H)
2D HSQC (Figure 2), δ ppm: 6.4-114.9 (signal 1), 7.18-128.6 (signal 4), 7.47 132.2 (signal 3), 7.62-129.7 (signal 5), 7.95-139.1 (signal 2).
H-H COSY: cross-peak signal 6.40-7.95 ppm, 7.19-7.62ppm, 7.47-7.19 ppm.
DOSY NMR (500 MHz, D₂O): 6.40-7.95 ppm: -11 to -12 log (m²/s), 1.90 4.70 ppm: -11 -12 log (m²/s)
UV/vis (0.05 %; H2O) λmax= 309 nm.

### Example 3. Activation of 2-Furyl acrylic acid by benzoyl chloride and reaction with Hyaluronan

Sodium Hyaluronate (1.0 g, 2.5 mmol) with a mean molecular weight of 8.0 x 10⁴g/mol was dissolved in 20 ml of distilled water. Isopropanol (10 ml) was slowly added to that solution to avoid a possible precipitation of the polysaccharide. Subsequently, 0.7 ml of triethylamine corresponding to 2 equivalents to HA dimer was added to the reaction mixture. In a second flask, 0.17 g (1.25 mmol) of 2-furyl-acrylic acid was dissolved in isopropanol (10 ml). After that 0.7 ml of TEA (corresponding to 2 equivalents to HA dimer) were added to the reaction, followed by the addition of 0.15 ml of benzoyl chloride (corresponding to 0.5 equivalents of HA dimer). The activation was carried out for 30 minutes at room temperature (25°C). After that, the solution was added to the first solution (containing HA). The reaction is carried out for 3 hours at room temperature. The reaction was finished by the addition of water, and a solution of sodium chloride. After that the product was precipitated with an excess of isopropanol (250 ml). The product was washed one time by using 50 ml of isopropanol (100%) after that 4 times using 50 ml of mixtures of isopropanol and water (85% v/v). Finally, the precipitate was washed three times more with 100% IPA and dried in an oven at 40°C. Yield of the reaction 92.8%, %, Mw of the derivative (SEC-MALLS) = 8.5x10⁴ g/mol and polydispersity= 1.5.

### Chemical description of the derivative

Degree of substitution was calculated to be 5.3 % according to the ¹H NMR.
1H NMR (500 MHz, D₂O), δ ppm, for linker: 6.40 (d, 1H), 7.18 (t, 1H), 7.47 (s, 1H), 7.62(d,1H), 7.95(d, 1H)
2D HSQC (Figure 2), δ ppm: 6.4-114.9 (signal 1), 7.18-128.6 (signal 4), 7.47-132.2 (signal 3), 7.62 129.7 (signal 5), 7.95-139.1 (signal 2).
H-H COSY: cross-peak signal 6.40-7.95 ppm, 7.19-7.62ppm, 7.47-7.19 ppm. DOSY NMR (500 MHz, D₂O): 6.40-7.95 ppm: -11 to -12 log (m2/s), 1.90-4.70 ppm: -11-12 log (m²/s)
UV/vis (0.05 %; H₂O λmax = 309 nm.

### Example 4. Activation of 2-Furyl acrylic acid by benzoyl chloride and reaction with Hyaluronan

Sodium Hyaluronate (30.0 g, 75 mmol) with a mean molecular weight of 8.3 x 10⁴g/mol was dissolved in 500 ml of distilled water. Isopropanol (250 mol) was slowly added to that solution to avoid a possible precipitation of the polysaccharide. Subsequently, 21 ml of triethylamine corresponding to 2 equivalents to HA dimer was added to the reaction mixture. In a second flask, 10.37 g (75 mmol) of 2-furyl-acrylic acid was dissolved in isopropanol (50 ml). After that 21 ml of TEA (corresponding to 2 equivalents to HA dimer) were added to the reaction, followed by the addition of 9 ml of benzoyl chloride (corresponding to 0.5 equivalents of HA dimer). The activation was carried out for 30 minutes at room temperature (25°C). After that, the solution was added to the first solution (containing HA). The reaction is carried out for 3 hours at room temperature. The reaction was finished by the addition of water, and a solution of sodium chloride. After that the product was precipitated with an excess of isopropanol (2500 ml). The product was washed one time by using 300 ml of isopropanol (100%) after that 4 times using 200 ml of mixtures of isopropanol and water (85% v/v). Finally, the precipitate was washed three times more with 100% IPA and dried in an oven at 40°C. Yield of the reaction 92.8%, Mw of the derivative (SEC-MALLS) = 8.9x10⁴ g/mol and polydispersity= 1.5

### Chemical description of the derivative

Degree of substitution was calculated to be 10 % according to the ¹H NMR.
1H NMR (500 MHz, D₂O), δ ppm, for linker: 6.40 (d, 1H), 7.18 (t, 1H), 7.47 (s, 1H), 7.62(d,1H), 7.95(d, 1H)
2D HSQC (Figure 2), δ ppm: 6.4-114.9 (signal 1), 7.18-128.6 (signal 4), 7.47-132.2 (signal 3), 7.62-129.7 (signal 5), 7.95-139.1 (signal 2).
H-H COSY: cross-peak signal 6.40-7.95 ppm, 7.19-7.62ppm, 7.47-7.19 ppm. DOSY NMR (500 MHz, D₂O): 6.40-7.95 ppm: -11 to -12 log (m2/s), 1.90-4.70 ppm: -11 -12 log (m²/s)
UV/vis (0.05 %; H₂O λmax = 309 nm.

### Example 5. Activation of 2-Furyl acrylic acid by benzoyl chloride and reaction with Hyaluronan

Sodium Hyaluronate (1.0 g, 2.5 mmol) with a mean molecular weight of 2.7 x 10⁵g/mol was dissolved in 20 ml of distilled water. Isopropanol (10 ml) was slowly added to that solution to avoid a possible precipitation of the polysaccharide. Subsequently, 0.7 ml of triethylamine corresponding to 2 equivalents to HA dimer was added to the reaction mixture. In a second flask, 0.346 g (2.5 mmol) of 2-furyl-acrylic acid was dissolved in isopropanol (10 ml). After that 0.7 ml of TEA (corresponding to 2 equivalents to HA dimer) were added to the reaction, followed by the addition of 0.30 ml of benzoyl chloride (corresponding to 0.5 equivalents of HA dimer). The activation was carried out for 30 minutes at room temperature (25°C). After that, the solution was added to the first solution (containing HA). The reaction is carried out for 3 hours at room temperature. The reaction was finished by the addition of water, and a solution of sodium chloride. After that the product was precipitated with an excess of isopropanol (250 ml). The product was washed one time by using 50 ml of isopropanol (100%) after that 4 times using 50 ml of mixtures of isopropanol and water (85% v/v). Finally, the precipitate was washed three times more with 100% IPA and dried in an oven at 40°C. Yield of the reaction 92.5%, Mw of the derivative (SEC-MALLS) = 2.9x10⁵ g/mol and polydispersity= 1.65

### Chemical description of the derivative

Degree of substitution was calculated to be 17 % according to the ¹H NMR.
1H NMR (500 MHz, D₂O), δ ppm, for linker: 6.40 (d, 1H), 7.18 (t, 1H), 7.47 (s, 1H), 7.62(d,1H), 7.95(d, 1H)
2D HSQC (Figure 2), δ ppm: 6.4-114.9 (signal 1), 7.18-128.6 (signal 4), 7.47-132.2 (signal 3), 7.62-129.7 (signal 5), 7.95-139.1 (signal 2).
H-H COSY: cross-peak signal 6.40-7.95 ppm, 7.19-7.62ppm, 7.47-7.19 ppm.
DOSY NMR (500 MHz, D₂O): 6.40-7.95 ppm: -11 to -12 log (m²/s), 1.90-4.70 ppm: 11 -12 log (m²/s)
UV/vis (0.05 %; H₂O) λmax= 309 nm.

### Example 6. Activation of 2-Furyl acrylic acid by benzoyl chloride and reaction with Hyaluronan

Sodium Hyaluronate (1.0 g, 2.5 mmol) with a mean molecular weight of 5.0 x 10⁵g/mol was dissolved in 17 ml of distilled water. Isopropanol (10 ml) was slowly added to that solution to avoid a possible precipitation of the polysaccharide. Subsequently, 1.0 ml of triethylamine corresponding to 3 equivalents to HA dimer was added to the reaction mixture. In a second flask, 0.173 g (1.25 mmol) of 2-furyl-acrylic acid was dissolved in isopropanol (10 ml). After that 1.0 ml of TEA (corresponding to 3 equivalents to HA dimer) were added to the reaction, followed by the addition of 0.15 ml of benzoyl chloride (corresponding to 0.5 equivalents of HA dimer). The activation was carried out for 30 minutes at room temperature (25°C). After that, the solution was added to the first solution (containing HA). The reaction is carried out for 3 hours at room temperature. The reaction was finished by the addition of water, and a solution of sodium chloride. After that the product was precipitated with an excess of isopropanol (250 ml). The product was washed one time by using 50 ml of isopropanol (100%) after that 4 times using 50 ml of mixtures of isopropanol and water (85% v/v). Finally, the precipitate was washed three times more with 100% IPA and dried in an oven at 40°C. Yield of the reaction 95.8%, Mw of the derivative (SEC-MALLS) = 5.2x10⁵ g/mol and polydispersity= 1.65

### Chemical description of the derivative

Degree of substitution was calculated to be 14 % according to the 1H NMR.
1H NMR (500 MHz, D₂O) δ ppm, for linker: 6.40 (d, 1H), 7.18 (t, 1H), 7.47 (s, 1H), 7.62(d,1H), 7.95(d, 1H)
2D HSQC (Figure 2), δ ppm: 6.4-114.9 (signal 1), 7.18-128.6 (signal 4), 7.47-132.2 (signal 3), 7.62-129.7 (signal 5), 7.95-139.1 (signal 2).
H-H COSY: cross-peak signal 6.40-7.95 ppm, 7.19-7.62ppm., 7.47-7.19 ppm.
DOSYNMR (500 MHz, D₂O): 6.40-7.95 ppm: -11 to -12 log (m²/s), 1.90-4.70 ppm: -11 -12 log (m²/s)
UV/vis (0.05 %; H₂O λmax = 309 nm.

### Example 7. Activation of 2-Furyl acrylic acid by benzoyl chloride and reaction with Hyaluronan

Sodium Hyaluronate (1.0 g, 2.5 mmol) with a mean molecular weight of 1.0 x 10⁶g/mol was dissolved in 20 ml of distilled water. Isopropanol (10 ml) was slowly added to that solution to avoid a possible precipitation of the polysaccharide. Subsequently, 0.7 ml of triethylamine corresponding to 2 equivalents to HA dimer was added to the reaction mixture. In a second flask, 0.17 g (1.25 mmol) of 2-furyl-acrylic acid was dissolved in isopropanol (10 ml). After that 0.7 ml of TEA (corresponding to 2 equivalents to HA dimer) were added to the reaction, followed by the addition of 0.15 ml of benzoyl chloride (corresponding to 0.5 equivalents of HA dimer). The activation was carried out for 30 minutes at room temperature (25°C). After that, the solution was added to the first solution (containing HA). The reaction is carried out for 3 hours at room temperature. The reaction was finished by the addition of water, and a solution of sodium chloride. After that the product was precipitated with an excess of isopropanol (250 ml). The product was washed one time by using 50 ml of isopropanol (100%) after that 4 times using 50 ml of mixtures of isopropanol and water (85% v/v). Finally, the precipitate was washed three times more with 100% IPA and dried in an oven at 40°C. Yield of the reaction 95.8%, Mw of the derivative (SEC-MALLS) = 1..0x10⁶ g/mol and polydispersity= 1.8

### Chemical description of the derivative

Degree of substitution was calculated to be 4.8 % according to the 1H NMR.
1H NMR (500 MHz, D₂O δ ppm, for linker: 6.40 (d, 1H), 7.18 (t, 1H), 7.47 (s, 1H), 7.62(d, 1H), 7.95(d, 1H)
2D HSQC (Figure 2), δ ppm: 6.4-114.9 (signal 1), 7.18-128.6 (signal 4), 7.47-132.2 (signal 3), 7.62-129.7 (signal 5), 7.95-139.1 (signal 2).
H-H COSY: cross-peak signal 6.40-7.95 ppm, 7.19-7.62ppm, 7.47-7.19 ppm. DOSY NMR (500 MHz, D₂O): 6.40-7.95 ppm: -11 to -12 log (m²/s), 1.90-4.70 ppm: -11-12 log (m²/s)
UV/vis (0.05 %; H₂O) λmax = 309 nm.

### Example 8. Activation of 2-Furyl acrylic acid by benzoyl chloride and reaction with Hyaluronan

Sodium Hyaluronate (1.0 g, 2.5 mmol) with a mean molecular weight of 1.6 x 10⁶g/mol was dissolved in 20 ml of distilled water. Isopropanol (10 ml) was slowly added to that solution to avoid a possible precipitation of the polysaccharide. Subsequently, 0.7 ml of triethylamine corresponding to 2 equivalents to HA dimer was added to the reaction mixture. In a second flask, 0.17 g (1.25 mmol) of 2-furyl-acrylic acid was dissolved in isopropanol (10 ml). After that 0.7 ml of TEA (corresponding to 2 equivalents to HA dimer) were added to the reaction, followed by the addition of 0.15 ml of benzoyl chloride (corresponding to 0.5 equivalents of HA dimer). The activation was carried out for 30 minutes at room temperature (25°C). After that, the solution was added to the first solution (containing HA). The reaction is carried out for 3 hours at room temperature. The reaction was finished by the addition of water, and a solution of sodium chloride. After that the product was precipitated with an excess of isopropanol (250 ml). The product was washed one time by using 50 ml of isopropanol (100%) after that 4 times using 50 ml of mixtures of isopropanol and water (85% v/v). Finally, the precipitate was washed three times more with 100% IPA and dried in an oven at 40°C. Yield of the reaction 95.8%, Mw of the derivative (SEC-MALLS) = 1.7x10⁶ g/mol and polydispersity= 1.7

### Chemical description of the derivative

Degree of substitution was calculated to be 4.2 % according to the ¹H NMR.
1H NMR (500 MHz, D₂O) δ ppm, for linker: 6.40 (d, 1H), 7.18 (t, 1H), 7.47 (s, 1H), 7.62(d,1H), 7.95(d, 1H)
2D HSQC (Figure 2), δ ppm: 6.4-114.9 (signal 1), 7.18-128.6 (signal 4), 7.47-132.2 (signal 3), 7.62-129.7 (signal 5), 7.95-139.1 (signal 2).
H-H COSY: cross-peak signal 6.40-7.95 ppm, 7.19 7.62ppm, 7.47-7.19 ppm.
DOSY NMR (500 MHz, D₂O): 6.40-7.95 ppm: -11 to -12 log (m²/s), 1.90-4.70 ppm: 11 -12 log (m²/s)
UV/vis (0.05 %; H₂O λmax = 309 nm.

### Example 9. Activation of 2-Furyl acrylic acid by benzoyl chloride and reaction with Hyaluronan

Sodium Hyaluronate (1.0 g, 2.5 mmol) with a mean molecular weight of 1.6 x 10⁶g/mol was dissolved in 20 ml of distilled water. Isopropanol (10 ml) was slowly added to that solution to avoid a possible precipitation of the polysaccharide. Subsequently, 0.7 ml of triethylamine corresponding to 2 equivalents to HA dimer was added to the reaction mixture. In a second flask, 0.259 g (1.88 mmol) or 0.75 equivalents of 2-furyl-acrylic acid was dissolved in isopropanol (10 ml). After that 0.7 ml of TEA (corresponding to 2 equivalents to HA dimer) were added to the reaction, followed by the addition of 0.21 ml of benzoyl chloride (corresponding to 0.75 equivalents of HA dimer). The activation was carried out for 30 minutes at room temperature (0°C). After that, the solution was added to the first solution (containing HA). The reaction is carried out for 3 hours at 60°C. The reaction was finished by the addition of water, and a solution of sodium chloride. After that the product was precipitated with an excess of isopropanol (250 ml). The product was washed one time by using 50 ml of isopropanol (100%) after that 4 times using 50 ml of mixtures of isopropanol and water (85% v/v). Finally, the precipitate was washed three times more with 100% IPA and dried in an oven at 40°C. Yield of the reaction 80%. The average molecular weight of the derivative was determined as 1.59x10⁶ and polydispersity of 1.52.

### Chemical description of the derivative

Degree of substitution was calculated to be 10 % according to the ¹H NMR.
1H NMR (500 MHz, D₂O), δ ppm, for linker: 6.40 (d, 1H), 7.18 (t, 1H), 7.47 (s, 1H), 7.62(d,1H), 7.95(d, 1H)
2D HSQC (Figure 2), δ ppm: 6.4-114.9 (signal 1), 7.18-128.6 (signal 4), 7.47-132.2 (signal 3), 7.62 129.7 (signal 5), 7.95-139.1 (signal 2).
H-H COSY: cross-peak signal 6.40-7.95 ppm, 7.19-7.62ppm, 7.47-7.19 ppm.
DOSY NMR (500 MHz, D₂O): 6.40-7.95 ppm: -11 to -12 log (m²/s), 1.90 4.70 ppm: 11 -12 log (m²/s)
UV/vis (0.05 %; H2O) λmax= 309 nm.

### Example 10. Activation of 2-Furyl acrylic acid by benzoyl chloride and reaction with Hyaluronan

Sodium Hyaluronate (1.0 g, 2.5 mmol) with a mean molecular weight of 1.8x10⁶ g/mol was dissolved in 10 ml of distilled water. Isopropanol (10 ml) was slowly added to that solution to avoid a possible precipitation of the polysaccharide. Subsequently, 0.7 ml of triethylamine corresponding to 2 equivalents to HA dimer was added to the reaction mixture. In a second flask, 0.34 g (2.5 mmol) of 2-furyl-acrylic acid was dissolved in isopropanol (10 ml). After that 0.7 ml of TEA (corresponding to 2 equivalents to HA dimer) were added to the reaction, followed by the addition of 0.29 ml of benzoyl chloride (corresponding to 1.0 equivalents of HA dimer). The activation was carried out for 30 minutes at room temperature (25°C). After that, the solution was filtered and added to the first solution (containing HA). The reaction is carried out for 2 hours at 40°C. The reaction was finished by the addition of water, and a solution of sodium chloride. After that the product was precipitated with an excess of isopropanol (250 ml). The product was washed one time by IPA, after that with a solution IPA: water (85% v/v, 4 x 50 ml). Finally, the precipitate was washed three times more with 100% IPA and dried in an oven at 40°C. Yield of the reaction 99%, Mw of the derivative (SEC-MALLS) was determined as 1.82x10⁶ and polydispersity= 1.61

### Chemical description of the derivative

Degree of substitution (corresponding to 12% of photoreactive groups)
**¹H** NMR (500 MHz, D₂O), δ ppm, for linker: 6.40 (d, 1H), 7.18 (t, 1H), 7.47 (s, 1H), 7.62(d,1H), 7.95(d, 1H)
**2D HSQC,** δ ppm: 6.4-114.9 (signal 1), 7.18-128.6 (signal 4), 7.47-132.2 (signal 3), 7.62-129.7 (signal 5), 7.95-139.1 (signal 2).
**H-H COSY:** cross-peak signal 6.40-7.95 ppm, 7.19-7.62ppm, 7.47-7.19 ppm.
**DOSY** NMR (500 MHz, D₂O): 6.40-7.95 ppm: -11 to -12 log (m²/s), 1.90-4.70 ppm: -11-12 log (m²/s)
UV/vis (0.05 %; H₂O λₘₐₓ= 309 nm.

### Example 11. Activation of 3-(2-Furyl) acrylic acid by 2, 4, 6-trichlorobenzoyl chloride and reaction with Hyaluronan

Sodium hyaluronate (30 g, 75 mmol) with a mean molecular weight of 1x10⁶ g/mol was dissolved in 1000 ml of distilled water. THF (1000 mol) were slowly added to that solution of avoid a possible precipitation of the polysaccharide. Subsequently, 21 ml of triethylamine (TEA) was added to the reaction mixture (corresponding to 2 equivalents to HA dimer). In a second flask, 5.18 (37.4 mmol) g of 2-furfuryl-acrylic acid was dissolved in THF (300 ml). After that 21 ml of TEA (corresponding to 2 equivalents to HA dimer) were added to the reaction, followed by the addition of 0.4 ml of 2, 4, 6-trichlorobenzoyl chloride (corresponding to 0.5 equivalents to HA dimer). The activation was carried out for 30 minutes. After that, the filtrated solution (soluble part) was added to the first solution (containing HA). The reaction is carried out for 3 hours at room temperature. The reaction was finished by the addition of water, and a solution of sodium chloride. The product was washed one time by IPA, after that with a solution IPA: water (85% v/v, 4 x 500 ml). Finally, the precipitate was washed three times more with 100% IPA and dried in an oven at 40°C. For all the reactions, "yield of the reaction" is reported. Yield of the reaction: 98% (average of three repetitions).

### Chemical description of the derivative

Degree of substitution (corresponding to 3% of photosensitive groups)
**¹H** NMR (500 MHz, D₂O), δ ppm, for linker: 6.40 (d, 1H), 7.18 (t, 1H), 7.47 (s, 1H), 7.62(d,1H), 7.95(d,1H)
**2D HSQC,** δ ppm: 6.4-114.9 (signal 1), 7.18-128.6 (signal 4), 7.47-132.2 (signal 3), 7.62-129.7 (signal 5), 7.95-139.1 (signal 2).
**H-H COSY:** cross-peak signal 6.40-7.95 ppm, 7.19-7.62 ppm, 7.47-7.19 ppm.
**DOSY** NMR (500 MHz, D₂O) 6.40-7.95 ppm: -11 to -12 logs (m²/s), 1.90-4.70 ppm: -11 -12 log (m²/s).
UV/vis (0.05 %; H2O) λmax = 309 nm.

### Example 12. Activation of 3-(2-Furyl) acrylic acid by 2, 4, 6-trichlorobenzoyl chloride and reaction with Hyaluronan

Sodium hyaluronate (30 g, 75 mmol) with a mean molecular weight of 8.5x10⁴ g/mol was dissolved in 600 ml of distilled water. THF (300 ml) were slowly added to that solution of avoid a possible precipitation of the polysaccharide. Subsequently, 21 ml of triethylamine (TEA) was added to the reaction mixture (corresponding to 2 equivalents to HA dimer). In a second vessel, 10.36 g (75 mmol) of 2-fuxfuryl-acrylic acid were dissolved in THF (300 ml). After that 21 ml of TEA (corresponding to 2 equivalents to HA dimer) were added to the reaction, followed by the addition of 12 ml of 2, 4, 6-trichlorobenzoyl chloride (corresponding to 1 equivalent to HA dimer). The activation was carried out for 30 minutes. After that, the filtrated solution (soluble part) was added to the first solution (containing HA). The reaction is carried out for 3 hours at room temperature. The reaction was finished by the addition of water, and a solution of sodium chloride. The product was washed one time by IPA, after that with a solution IPA: water (85% v/v, 4 x 500 ml). Finally, the precipitate was washed three times more with 100% IPA and dried in an oven at 40°C. For all the reactions, "yield of the reaction" is reported. That means the percentage gain/loss of the product compared to the amount of weight HA-Na in the reaction. A yield of the reaction was 91% (average of three reactions). Determination of Molecular weight by SEC-MALLS is 9.0x10⁴ g/mol and polydispersity= 1.52

### Chemical description of the derivative

Degree of substitution (corresponding to 6.2% of photosensitive groups)
**¹H** NMR (500 MHz, D₂O) δ ppm, for linker: 6.40 (d, 1H), 7.18 (t, 1H), 7.47 (s, 1H), 7.62(d,1H), 7.95(d, 1H)
**2D HSQC,** δ ppm: 6.4-114.9 (signal 1), 7.18-128.6 (signal 4), 7.47-132.2 (signal 3), 7.62-129.7 (signal 5), 7.95-139.1 (signal 2).
**H-H COSY:** cross-peak signal 6.40-7.95 ppm, 7.19-7.62ppm, 7.47-7.19 ppm.
**DOSY** NMR (500 MHz, D₂O): 6.40-7.95 ppm: -11 to -12 log (m²/s), 1.90-4.70 ppm: -11-12 log (m²/s)
UV/vis (0.05 %; H2O) λmax = 309 nm.

### Example 13. Activation of 3-(2-Furyl) acrylic acid by benzoyl chloride and reaction with Hyaluronan

Sodium hyaluronate (30 g, 75 mmol) with a mean molecular weight of 8.5x10⁴ g/mol was dissolved in 600 ml of distilled water. THF (300 ml) were slowly added to that solution of avoid a possible precipitation of the polysaccharide. Subsequently, 31ml of triethylamine (TEA) was added to the reaction mixture (corresponding to 3 equivalents to HA dimer). In a second flask, 20.73 g of 2-furfuryl-acrylic acid corresponding to 2 equivalents of HA dimer were dissolved in THF (300 ml). After that 31 ml of TEA (corresponding to 3 equivalents to HA dimer) were added to the reaction, followed by the addition of 0.2 ml of benzoyl chloride (corresponding to 2 equivalents to HA dimer). The activation was carried out for 30 minutes at 0°C. After that, the filtrated solution (soluble part) was added to the first solution (containing HA). The reaction is carried out for 3 hours at 0°C. The reaction was finished by the addition of water, and a solution of sodium chloride. After that the product was precipitated with an excess of isopropanol (1000 ml). The product was washed one time by IPA, after that with a solution IPA: water (85% v/v, 4 x 500 ml). Finally, the precipitate was washed three times more with 100% IPA and dried in an oven at 40°C. Determination of Molecular weight by SEC-MALLS is 9.0x10⁴ g/mol and polydispersity= 1.52.

### Chemical description of the derivative

Degree of substitution (corresponding to 18±2% of photosensitive groups)
**¹H** NMR (500 MHz, D₂O), δ ppm, for linker: 6.40 (d, 1H), 7.18 (t, 1H), 7.47 (s, 1H), 7.62(d,1H), 7.95(d, 1H)
**2D HSQC,** δ ppm: 6.4-114.9 (cross peak, signal 1), 7.18-128.6 (signal 4), 7.47-132.2 (signal 3), 7.62-129.7 (signal 5), 7.95-139.1 (signal 2).
**H-H COSY:** cross-peak signal 6.40-7.95 ppm, 7.19-7.62ppm, 7.47-7.19 ppm.
**DOSY** NMR (500 MHz, D₂O): 6.40-7.95 ppm: -11 to -12 log (m²/s), 1.90-4.70 ppm: -11 -12 log (m²/s)
UV/vis (0.05 %; H₂O) λₘₐₓ = 309 nm.

### Example 14. Activation of 3-(2-Furyl) acrylic acid by 2, 4, 6-trichlorobenzoyl chloride and addition to Hyaluronan, wherein reaction is catalyzed by DMAP

Sodium hyaluronate (30 g, 75 mmol) with a mean molecular weight of 8.5x10⁴ g/mol was dissolved in 600 ml of distilled water. THF (300 ml) were slowly added to that solution of avoid a possible precipitation of the polysaccharide. Subsequently, 31 ml of triethylamine (TEA) was added to the reaction mixture (corresponding to 3 equivalents to HA dimer), followed by 0,458 g of 4-Dimethyl aminopyridine (DMAP), corresponding to 0.05 equivalents of HA dimer. In a second flask, 31 g of 2-furfuryl-acrylic acid corresponding to 3 equivalents of HA dimer were dissolved in THF (300 ml). After that 31 ml of TEA (corresponding to 3 equivalents to HA dimer) were added to the reaction, followed by the addition of 35.1 ml of 2, 4, 6 trichlorobenzoyl chloride (corresponding to 3 equivalents to HA dimer). The activation was carried out for 30 minutes. After that, the filtrated solution (soluble part) was added to the first solution (containing HA). The reaction is carried out for 3 hours at room temperature. The reaction was finished by the addition of water, and a solution of sodium chloride. After that the product was precipitated with an excess of isopropanol (1000 ml). The product was washed one time by IPA, after that with a solution IPA: water (85% v/v, 4 x 250 ml). Finally, the precipitate was washed three times more with 100% IPA and dried in an oven at 40°C. For all the reactions, we have reported "yield of the reaction". That means the percentage gain/loss of the product compared to the amount of weight HA-Na in the reaction. Yield of the reaction: 99%. Due to the high viscosity of the derivative, the molecular weight cannot be determined by SEC-MALLS methodology due to possible cross-linking.

### Chemical description of the derivative

Degree of substitution (corresponding to 30-40% of photosensitive groups)
**¹H** NMR (500 MHz, D₂O), δ ppm, for linker: 6.40 (d, 1H), 7.18 (t, 1H), 7.47 (s, 1H), 7.62(d,1H), 7.95(d,1H)
**2D HSQC,** δ ppm: 6.4-114.9 (signal 1), 7.18-128.6 (signal 4), 7.47-132.2 (signal 3), 7.62-129.7 (signal 5), 7.95-139.1 (signal 2).
**H-H COSY:** cross-peak signal 6.40-7.95 ppm, 7.19-7.62ppm, 7.47-7.19 ppm.
**DOSY** NMR (500 MHz, D₂O); 6.40-7.95 ppm: -11 to -12 log (m²/s), 1.90-4.70 ppm: -11 -12 log (m²/s)
UV/vis (0.05 %; H₂O λₘₐₓ= 309 nm.

### Example 15. Activation of 2-thienyl acrylic acid by 2, 4, 6-trichlorobenzoyl chloride and reaction with Hyaluronan

Sodium Hyaluronate (5.0 g, 12.5 mmol) with a mean molecular weight of 1.01x10⁵ g/mol was dissolved in 100 ml of distilled water. THF (50 ml) were slowly added to that solution of avoid a possible precipitation of the polysaccharide. Subsequently, 3.5 ml of triethylamine corresponding to 2 equivalents to HA dimer was added to the reaction mixture. In a second flask, 1.0 g of 2-thienyl-acrylic acid was dissolved in THF (50 ml). After that 2.5 ml of TEA (corresponding to 3 equivalents to HA dimer) were added to the reaction, followed by the addition of 0.95 ml of benzoyl chloride (corresponding to 0.5 equivalents of HA dimer). The activation was carried out for 30 minutes at 60°C. After that, the solution was filtered and added to the solution containing HA. The reaction mixture was stirred out for 3 hours at 60°C. The reaction was finished by the addition of water, and a solution of sodium chloride. After that the product was precipitated with an excess of isopropanol (250 ml). The product was washed one time by IPA, after that with a solution IPA: water (85% v/v, 4 x 50 ml). Finally, the precipitate was washed three times more with 100% IPA and dried in an oven at 40°C. Yield of the reaction 92.18%, Mw of the derivative (SEC-MALLS) = 1.08x10⁵ and polydispersity= 1.482

### Chemical description of the derivative

Degree of substitution (corresponding to 5% of photosensitive groups)
**¹H** NMR (500 MHz, D₂O), δ ppm, for linker: 6.40 (d, 1H), 7.18 (t, 1H), 7.47 (s, 1H), 7.62(d,1H), 7.95(d,1H)
**2D HSQC,** δ ppm: 6.4-114.9 (signal 1), 7.18-128.6 (signal 4), 7.47-132.2 (signal 3), 7.62-129.7 (signal 5), 7.95-139.1 (signal 2).
**H-H COSY:** cross-peak signal 6.40-7.95 ppm, 7.19-7.62ppm, 7.47-7.19 ppm.
**DOSY** NMR (500 MHz, D₂O): 6.40-7.95 ppm: -11 az -12 log (m²/s), 1.90-4.70 ppm: -11 -12 log (m²/s)
UV/vis (0.05 %; H₂O) λₘₐₓ = 314 nm.

### Example 16. Activation of 3-(2-Thienyl) acrylic acid by 2, 4, 6-trichlorobenzoyl chloride and addition to Hyaluronan, wherein reaction is catalyzed by DMAP

Sodium Hyaluronate (5.0 g, 12.5 mmol) with a mean molecular weight of 1.01x10⁵ g/mol was dissolved in 100 ml of distilled water. THF (50 ml) were slowly added to that solution of avoid a possible precipitation of the polysaccharide. Subsequently, 5.2 ml of triethylamine corresponding to 3 equivalents to HA dimer was added to the reaction mixture, followed by 0.076 g of 4-dimethyl aminopyridine (DMAP). In a second flask, 6.36 g of 2-thienyl-acrylic acid was dissolved in THF (50 ml). After that 5.2 ml of TEA (corresponding to 3 equivalents to HA dimer) were added to the reaction, followed by the addition of 5.8 ml of 2, 4, 6 trichlorobenzoyl chloride (corresponding to 3 equivalents of HA dimer). The activation was carried out for 30 minutes at room temperature (25°C). After that, the solution was filtered and added to the first solution (containing HA). The reaction is carried out for 3 hours at room temperature. The reaction was finished by the addition of water, and a solution of sodium chloride. The product was washed one time by IPA, after that with a solution IPA: water (85% v/v, 4 x 50 ml). Finally, the precipitate was washed three times more with 100% IPA and dried in an oven at 40°C. Yield of the reaction 95%. Due to the high viscosity of the derivative, the molecular weight cannot be determined by SEC-MALLS methodology

### Chemical description of the derivative

Degree of substitution (corresponding to 30% of photosensitive groups)
**¹H** NMR (500 MHz, D₂O), δ ppm, for linker: 6.40 (d, 1H), 7.18 (t, 1H), 7.47 (s, 1H), 7.62(d,1H), 7.95(d, 1H)
**2D HSQC,** δ ppm: 6.4-114.9 (signal 1), 7.18-128.6 (signal 4), 7.47-132.2 (signal 3), 7.62-1.29.7 (signal 5), 7.95-139.1 (signal 2).
**H-H COSY:** cross-peak signal 6.40-7.95 ppm, 7.19-7.62ppm, 7.47-7.19 ppm.
**DOSY** NMR (500 MHz, D₂O): 6.40-7.95 ppm: -11 až -12 log (m²/s), 1.90-4.70 ppm: -11 -12 log (m²/s)
UV/vis (0.05 %; H₂O λₘₐₓ = 314 nm.

### Example 17. Activation of 3-(2-Furanyl) acrylic acid by 4-methoxybenzoyl chloride and reaction with Hyaluronan

1.0 g of Hyaluronic acid sodium salt (2.5 mmol, 8.5x10⁴ g/mol) was dissolved in 20 ml of distilled water. Tetrahydrofurane (10 ml) was slowly added to that solution followed by 0.7 ml of triethylamine (5 mmol). In a second flask, 0.17 g of Furyl acrylic acid (1.25 mmol) was solubilized in 10 ml of tetrahydrofurane, after 5 minutes of mixing 0.7 ml of triethylamine (TEA) were added, followed by 0.17 ml of p-methoxy benzoyl chloride (1.25 mmol). The activation was allowed to occur for 30 minutes. After that, the second solution was added to the first one (containing Hyaluronic acid). The mixture was allowed to react for 2 hours at room temperature. After that, 200 ml of absolute isopropanol were added, followed by 1 ml of super saturated solution of sodium chloride. The product was washed out 3 times by 100% isopropanol, and 4 times with a solution of isopropanol: water (85:15) and 2 times once more with 100% isopropanol. The reaction product was dried in an oven overnight and characterized. Determination of Molecular weight by SEC-MALLS is 9.5 x10⁴ g/mol and polydispersity= 1.52

### Chemical description of the derivative

Degree of substitution (corresponding to 1% of photosensitive groups)
¹H NMR (500 MHz, D₂O), δ ppm, for linker: 6.40 (d, 1H), 7.18 (t, 1H), 7.47 (s, 1H), 7.62(d,1H), 7.95(d, 1H)
UV/vis (0.05 %; H₂O) λₘₐₓ = 309 nm.

### Example 18. Activation of 3-(2-Furanyl) acrylic acid by 4-nitrobenzoyl chloride and reaction with Hyaluronan

1.0 g of Hyaluronic acid sodium salt (2.5 mmol, 5x10³ g/mol) was dissolved in 20 ml of distilled water. To that solution 10 ml of tetrahydrofurane were slowly added followed by 0.7 ml of triethylamine (5 mmol). In a second flask, 0.17 g of Furyl acrylic acid (1.25 mmol) was solubilized in 10 ml of tetrahydrofurane, after 5 minutes of mixing 0.7 ml of triethylamine (TEA) were added, followed by 0.23 g of p-nitro-benzoyl chloride (1.25 mmol). The activation was allowed to occur for 30 minutes. After that, the second solution was added to the first one (containing Hyaluronic acid). The mixture was allowed to react for 2 hours at room temperature. After that, 200 ml of absolute isopropanol were added, followed by 1 ml of super saturated solution of sodium chloride. The product was washed out 3 times by absolute isopropanol, and 4 times with a solution of isopropanol: water (85:15) and 2 times with absolute isopropanol. The reaction product was dried in an oven overnight and characterized. Determination of Molecular weight by SEC-MALLS is 5.5x10³ g/mol and polydispersity= 1.52

### Chemical description of the derivative

Degree of substitution (corresponding to 1% of photosensitive groups)
**¹H** NMR (500 MHz, D₂O), δ ppm, for linker: 6.40 (d, 1H), 7.18 (t, 1H), 7.47 (s, 1H), 7.62(d,1H), 7.95(d, 1H).
UV/vis (0.05 %; H₂O) λₘₐₓ = 309 nm.

### Example 19 Activation of Trans-3-(3-Pyridyl) acrylic acid and reaction with Hyaluronan

1.0 g of Hyaluronic acid sodium salt (2.5 mmol, 8.5x10⁴ g/mol) was dissolved in 20 ml of distilled water. Isopropanol (10 ml) was slowly added to that solution, followed by 0.7 ml of triethylamine (5 mmol). In a second flask, 0.19 g of *trans*-3-(3-Pyridyl) acrylic acid (1.25 mmol) was solubilized in 10 ml of isopropanol, after 5 minutes of mixing 0.8 ml of Diisopropylethylamine (DIPEA) were added, followed by 0.15ml of benzoyl chloride (1.25 mmol). The activation was allowed occur for 30 minutes. After that, the second solution was added to the first one (containing Hyaluronic acid). The mixture was allowed to react for 2 hours at room temperature. After that, 200 ml of absolute isopropanol were added, followed by 1 ml of super saturated solution of sodium chloride. The product was washed out 3 times by absolute isopropanol, and 4 times with a solution of isopropanol: water (85:15) and 2 times with absolute isopropanol. The reaction product was dried in an oven overnight and characterized. Determination of Molecular weight by SEC-MALLS is 9.0x10⁴ g/mol and polydispersity= 1.6
Degree of substitution was determined as 9% (corresponding to the amount of photosensitive groups). ¹H NMR spectra is depicted in Figure 2.

### Chemical description of the derivative

**¹H** NMR (500 MHz, D₂O) δ ppm, for linker: 6.74 (d, 1H, J=15.9), 7.53 (m, 1H), 7.70 (d,1H, J=17.5), 8.09 (m, 1H), 8.55 (m, 1H), 8.76 (m, 1H).
UV/vis (0.05 %; H₂O) λₘₐₓ = 263 nm.

### Example 20. Activation of 3-(4-imidazolyl) acrylic acid and reaction with Hyaluronan

1.0 g of Hyaluronic acid sodium salt (2.5 mmol, 8.5x10⁴ g/mol) was dissolved in 20 ml of distilled water. Isopropanol (10 ml) was slowly added to that solution, followed by 0.7 ml of triethylamine (5 mol). In a second flash, 0.173 g of 3-(4-imidazolyl) acrylic acid (1.25 mmol) was solubilized in 10 ml of isopropanol, after 5 minutes of mixing 0.7 ml of triethylamine (TEA) were added, followed by 0.15ml of benzoyl chloride (1.25 mmol). The activation was allowed to occur for 30 minutes. After that, the second solution was added to the first one (containing Hyaluronic acid). The mixture was allowed to react for 2 hours at room temperature. After that, 200 ml of absolute isopropanol were added, followed by 1 ml of super saturated solution of sodium chloride. The product was washed out 3 times by absolute isopropanol, and 4 times with a solution of isopropanol: water (85:15) and 2 times with absolute isopropanol. The reaction product was dried in an oven overnight and characterized.

### Chemical description of the derivative

Degree of substitution (corresponding to 5% of photosensitive groups)
**¹H** NMR (500 MHz, D₂O), δ ppm, for linker: 6.40 (m, 1H), 7.30 (m, 1H), 7.38 m, 1H), 7.52 (2H), 7.71 (m, 1H), 8.0 (1H).
UV/vis (0.05 %; H₂O) λₘₐₓ = 286 nm.

### Example 21. Activation of phenyl acrylic acid by benzoyl chloride and reaction with Hyaluronic acid

1.0 g of Hyaluronic acid sodium salt (2.5 mmol, 5x10³ g/mol) was dissolved in 10 ml of distilled water. Isopropanol (5 ml) was slowly added to that solution, followed by 0.7 ml of triethylamine (5 mmol). In a second flask, 0.37 g of phenyl acrylic acid (2.5 mmol) was solubilized in 5 ml of isopropanol, after 5 minutes of mixing 0.7 ml of triethylamine (5 mmol) were added, followed by 0.29 ml of benzoyl chloride (2.5 mmol). The activation was carried out for 30 minutes. After that, the second solution was added to the first one (containing Hyaluronic acid). The mixture was allowed to react for 2 hours at room temperature. After that, 200 ml of absolute isopropanol were added, followed by 1 ml of super saturated solution of sodium chloride. The product was washed out 3 times by absolute isopropanol, and 4 times with a solution of isopropanol: water (85:15) and 2 times with absolute isopropanol. The reaction product was dried in an oven overnight and characterized.

### Chemical description of the derivative

Degree of substitution (corresponding to 9% of photosensitive groups)
¹H NMR (500 MHz, D₂O), δ ppm, for linker: 6.64 (d, J= 16.4, 1H), 7.50 (bs, 3H), 7.70(m, 2H), 7.52 (2H), 7.82 (d, J=16.5, 1H).
UV/vis (0.05 %; H₂O) λₘₐₓ = 281 nm.

### Example 22. Obtention of nanofibers comprising native HA and ester derivatives of HA

A solution is prepared by dissolving the derivatives described in Table 1. Thus, the solutions having a concentration of the particular ester derivative of HA of previously described examples and polyethylene oxide was 5% (w/v) in water, keeping a ratio of 80:20 (HA/PEO of 4x 10⁵ g/mol) were prepared and used. These nanofiber layers were formed from those solutions. The electrospinning was performed using a wide multi-nozzle using the device 4spin® (CONTIPRO, Biotech s.r.o.). However, the production of the nanofibers is not limited to that device. The nanofibers were produced by using a collecting electrode at a distance of 20 cm and varying electric voltage from 60-80kV. And a surface tension of the solution (58.1 mN. m⁻¹). The conductivity and viscosity of the produced solutions are resumed in Table 1. Nanofibers of different diameter size averages from 99 to 150 nm can be obtained (see Table 1). The microstructure of the nanofibers was characterized by SEM (As a selected example, the microstructure of the derivative with degree of substitution 5% corresponding to preceding examples are shown on Figures 3 and 4, respectively). The table contains as well a reference sample of nanofibers obtained in based of Native Hyaluronic acid (on Entry 1) and the ester derivatives prepared in previously described examples which formed (Examples 2,3,4,12,13) or non-formed nanofibers (described in Examples 14, 16). The last two examples are used the toxic catalyst DMAP that induces self cross-linking of the derivatives before electrospinning. The nanofibers were at least prepared three independent times.

**Table 1. Processing parameters of HA and Sodium 3-(2-Furanyl) acryloyl Hyaluronate as described in proceeding Examples.**

| **Examples** | **DS_{Photo}(%)** | **Viscosity** | **Electrical conductivity** µS cm⁻¹ | **Average fiber diameter (nm)** |
|---|---|---|---|---|
| HA+PEO (400,000) | 0 | 3.54 | 6.2-7.3 | 109.1-121.68 |
| **2** | 4.2±2 | 1.25 | 4.1 | 133.1 |
| **3** | 5.3±2 | 1.40 | 4.9 | 120.7± 10 |
| **12** | 6.2±2 | 1.54 | 4.5 | 127.4 |
| **4** | 10±2 | 1.79 | 4.9 | 126.5±3.5 |
| **13** | 18±2 | 1.85 | 4.4 | 141.5±15.9 |
| **14,16** | 30-40 | 3.5 | 4.2 | no |

| | | | | |
|---|---|---|---|---|
| **no= non obtained** | | | | |

### Example 23. Obtention of nanofibers comprising the ester derivative of HA prepared in the Example 3 and different composition of polyethylene oxide

Spinning solution was prepared by dissolving the derivatives of Example 3, characterized by a molecular weight of 8.6 x10⁴ g/mol and a polyethylene oxide of molecular weight of 6x10⁵g/mol in water. The solution was prepared by stirring for 8 hours. The used concentration was 9.7% (w/w) and the weight ratio of HA/PEO was (74/26). The spinning was carried out by electrospinning from a wide multi-nozzle in the device 4Spin® from Contipro Biotech s.r.o, wherein the collecting electrode had an electrical voltage between (60 to 80) kV, the solution had shown a surface tension of solution of 55.1 mN.m-1 and a conductivity of 7.35 mS.cm⁻¹, and viscosity of 6.72 Pa s. The average diameter of electrostatically spun nanofibers detected by scanning electron microscopy and image analysis was about 261 nm.

### Example 24. Obtention of nanofibers comprising the ester derivative of HA in the Example 3 and polyvinyl alcohol

A solution is prepared by dissolving the derivatives described in Example 3. Thus, a concentration of 5% (w/w in water and polyvinyl alcohol (PVA) of molecular weight 4x 10⁵ g/mol keeping a ratio of 80:20 (HA/PVA). These nanofiber layers were formed from those solutions. The electrospinning was performed using a wide multi-nozzle using the device 4spin® (CONTIPRO, Biotech s.r.o.). However, the production of the nanofibers is not limited to that device. The nanofibers were produced by using a collecting electrode at a distance of 20 cm and varying electric voltage from 60-80kV. And a surface tension of the solution (58.1 mN. m-1) and a conductivity of 7.35 mS.cm⁻¹, and viscosity of 6.5 Pa s. The microstructure of electrostatically spun nanofibers is shown in Figure 5

### Example 25. Obtention of nanofibers comprising the ester derivative of HA in the Example 3 and polyvinyl pyrrolidone

A solution is prepared by dissolving the derivatives described in Example 3 in a mixture 1:1 of water and ethanol. Thus, a concentration of 5% (w/w in water and polyvinylpyrrolidone (PVP) of molecular weight 4x 10⁵ g/mol keeping a ratio of 80:20 (HA/PVP). These nanofiber layers were formed from those solutions. The electrospinning was performed using a wide multi-nozzle using the device 4spin® (CONTIPRO, Biotech s.r.o.). However, the production of the nanofibers is not limited to that device. The nanofibers were produced by using a collecting electrode at a distance of 20 cm and varying electric voltage from 60-80kV.

### Example 26. Obtention of nanofibers comprising ester derivatives of HA and PEO

A solution is prepared by dissolving the derivatives described in Example 15 or with the derivative described in Example 20. Thus, the solutions having a concentration of this particular ester derivative of HA of previously described examples and polyethylene oxide was 5% (w/v) in water, keeping a ratio of 80:20 (HA/PEO of 4x 105 g/mol) were prepared and used. These nanofiber layers were formed from those solutions. The electrospinning was performed using a wide multi-nozzle using the device 4spin® (CONTIPRO, Biotech s.r.o.). However, the production of the nanofibers is not limited to that device. The nanofibers were produced by using a collecting electrode at a distance of 20 cm and varying electric voltage from 60-80kV.

| **Example** | **DSₚₕₒₜₒ (%)** | **Viscosity Pa.s** | **Electrical conductivity** µS cm⁻¹ | **Average fiber diameter** |
|---|---|---|---|---|
| 15 | 5±2 | 1.29 | 4.8 | 99.58 ± 10.9 |
| 20 | 5±2 | 1.35 | 5.2 | 100.22 ± 4.4 |

### Example 27. UV-induced cross-linking of nanofibers

Different specimens of nanofiber mats were prepared as described in Example 22 and exposed to UV irradiation inside an oven designed by Eppendorf Czech & Slovakia s.r.o under the designation CL-1000 (302 nm). This device provides uniform UV radiation in the UV/Vis region of the spectrum is usually from 280 to 315 nm with a constant power (approximately 6.75 mW.cm⁻²). The maximum value of the relative radiation energy is declared at a wavelength of 302 nm. UV radiation is emitted by five parallel gas-discharge tubes, each of which has an output of 8 watts located below the upper wall. The radiation dose was E = 24300 µJ.cm⁻²) and at a constant power (6.75 mW.cm⁻²). The nanofibers are exposed for 5, 10, 20, 30 and 60 minutes for 10 or 60 minutes. The microstructure of the nanofibers after swelling in PBS corresponding to example 3 on the table, 1 is shown in Figure 6.

### Example 28. Water content calculation of the nanofibrous material obtained thereof

Samples of circular shape with a mean diameter of 5.0 cm were cut out with a special tool. The area of the samples was determined as 0.0078 square meters. To be more precise, the initial amount of derivative was weighted to have in all the cases circa 0.2 g or 0.5g per sample respectively. Thereafter, the samples were immersed in demineralized water or saline solution with a concentration of 0.9 %(w/v) of sodium chloride dissolved in water. The samples were immersed in a definite volume of 30 ml for 0.5 g sample and 12 ml in the case of 0.2 g of sample for 5 minutes. After soaking the material, the sample is filtered through a mesh (pore size of 90-160 µm). The material was then weighted, so that the amount of absorbed water or saline solution was determined (wₛ). Water absorption is determined by the formula Qw= (w_{d}-wₛ/w_{d}) x 100; Where w_{d} and wₛ are the weight of the dried sample and swollen, respectively.

| Swelling media | Initial weight (g) | cross-linking time (m) | Water absorption Q_{w} |
|---|---|---|---|
| H₂O | 0.2 | 10 | 20.0 |
| H₂O | 0.5 | 10 | 20.6 |
| 0.9% (w/v) NaCl | 0.2 | 10 | 10.2 |
| 0.9%(w/v)NaCl | 0.2 | 60 | 17.1 |

### Example 29.Cell viability assay of Sodium Furan-2-acrylate ester of Hyaluronan

The biocompatibility of the esters of hyaluronic acid prepared as described in previously described examples (1-9) were examined using on 3T3-NIH cell lines. Thus, 0.1 (w/v) % solution of the derivative was dissolved in complete cell culture media thorough mixing (overnight). After that, the solution was filtered thorough sterile filtration device (0.22 µm), forming final concentrations of the derivative such as 1, 0.5 and 0.1 mg/ml. 3000 cells lines (3T3-NIH) were seeded into wells of 96-well test plates. The cells were cultured for 24 hours before treated with the tested solutions. Cell viability was measured 0, 24, 48, 72 hours after treatment using the 3-(4,5-dimethylthiazol-2-yl)-2.5-diphenyl tetrazolium bromide (MTT) assay. Briefly, 20 µL of MTT stock solution (5 mg/ml) was added to 200 µl of cell culture medium in each well. The plates were incubated at 37°C in a cell culture incubator for 2.5 hours. MTT solution was removed and added 220 µl of lysis solution. Cells were lysed for 30 minutes at room temperature on an orbital shaker and the optical density was measured by Microplate reader VERSAmax at 570 nm. The experiment design was completed with a set of control cells cultured in common media without treatment and blank samples. MTT test was utilized to obtain basic information about cell metabolism and proliferation. Thus, these derivatives were found to be non-cytotoxic up to concentration of 1000 µg/ml.

### Example 30. Evaluation of photo-cytotoxicity for the derivative Sodium Furan-2-acrylate ester of Hyaluronan

10,000 cells (3T3-NIH) were seeded into wells of 96-welltest plates. The cells were cultured for 24 hours before treatment with the tested solutions in full medium. After that, the cells were rinsed with PBS and incubated with for one hour with the derivative previously dissolved in PBS (1-1000ug/ml). The cells with derivative were irradiated with a dose of 0.1 J/cm2 UVA/UVB with lamp (Oriel Instruments). Power of the lamp was determined using a photometer PMA 2100 (Solar light Co.). 10 minutes after exposure the supernatant was sucked out and full media was added. Cell viability was measured 24 hours after irradiation and using the MTT method. The experiment design was completed with a set of control cells cultured in common media without treatment and UV irradiation and also with positive and negative control. Phototoxicity assay can be utilized to identify a possible phototoxic effect of the derivative Sodium Furanyl-acrylic ester of Hyaluronan. The phototoxicity assay was repeated minimally four times and the mean (percentage) relative to the control and standard error of the means (SEMs) are calculated. Optical density was measured and the percentage relative to the control was calculated as a function of time.

### Example 31. Cell viability assay of Sodium thiophenyl-acrylic ester of Hyaluronan

Sodium Thiophenyl-acrylic ester of Hyaluronan prepared as described in above examples and its biocompatibility was examined using on 3T3-NIH cell lines. Thus, a 0.1 (w/v) % solution of the derivative was dissolved in complete cell culture media thorough mixing (overnight). The method described in Example 7 was performed as well for this derivative. These derivatives were also found to be non-cytotoxic up to concentration of 1000 µg/mL.

### Example 32. Evaluation of photo-cytotoxicity for the derivative sodium thiophenyl-acrylic ester of Hyaluronan

Sodium thiophenyl-acrylic ester of Hyaluronan prepared as described in above examples and its biocompatibility was examined using 3T3-NIH cell lines and photocytoxicity test. The method described in Example 9 was performed as well for this derivative. This derivative was also found to be non-photocytoxic. All derivatives have been tested as non-cytotoxic and non-photocytoxic for 3T3-NIH cell lines. Thus, the viability of cells after their treatment has not significantly changed in whole monitored interval.

## Claims

1. Nanofibers based on ester derivatives of hyaluronic acid and its salt **characterized in that** they comprise a photocurable ester derivative of hyaluronic acid or its salt of the general formula IV 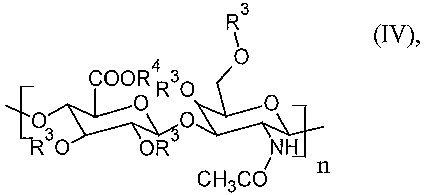
wherein n is integer in the range of 1 to 5000 dimers,
R³ are independently H or COCHCHR¹ wherein R¹ is 5- or 6-membered aryl or 5- or 6-membered heteroaryl having at least one or more identical or different heteroatoms selected from a group comprising N, O, S, provided that at least one R³ of the derivative is COCHCHR¹;
R⁴ is H⁺ or pharmaceutically acceptable salt;
and at least one carrying polymer.

2. Nanofibers according to Claim 1 **characterized in that** R¹ is selected from a group comprising phenyl, furyl, furfuryl, thienyl, thiofenyl, pyridyl or imidazoyl;
R⁴ is selected from a group comprising any of ions of alkali metals or ions of alkaline-earth metals, preferably Na⁺, K⁺, Mg²⁺ or Li⁺.

3. Nanofibers according to any of Claims 1 to 2 **characterized in that** the amount of COCHCHR¹ in the photocurable ester HA-derivative is from 0.1 to 20 % with respect to 100 dimers of hyaluronic acid or its salt, preferably 5 to 10 %, more preferably 5%.

4. Nanofibers according to any of Claims 1 to 3 **characterized in that** the carrying polymer is selected from a group comprising polyvinyl alcohol, polyacrylic acid, polyethylene oxide, polyvinyl pyrrolidone; the amount of the carrying polymer being in the range of 50% (w/w) to 99% (w/w), preferably in the range of 70 to 90% (w/w), most preferably 80% (w/w)..

5. The nanofibers of any of Claims 1 to Claim 4 **characterized in that** it comprises additionally biologically compatible polymer preferably carboxymethyl cellulose, gelatin, chitosan, polycaprolactone, polylactic acid, polyamide, polyurethane, poly-(lactide-co-glycolic) acid; and their mixture or their copolymers.

6. The nanofibers of any of Claims 1 to Claim 5 **characterized in that** the average diameter thereof is between 1 to 500 nm, preferably 99 to 300 nm.

7. The nanofibers according to any of Claims 1 to Claim 6 **characterized in that** they are in the form of gel.

8. A method of synthesis of nanofibers according to any of Claims 1 to 7 **characterized in that** α,β-unsaturated substituted acrylic acid of a general formula I 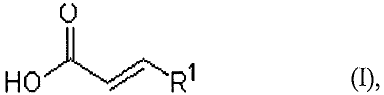 wherein
R¹ is is 5- or 6-membered aryl or 5- or 6-membered heteroaryl having at least one or
more identical or different heteroatoms selected from the group comprising N, O or S; is activated with substituted or non-substituted benzoyl chloride or its derivatives of a general formula II 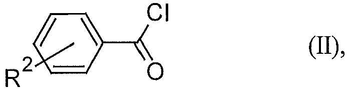 wherein R² is one or more substituents selected from a group comprising H, NO₂, -COOH,
halides, C₁-C₆alkylkoxy, preferably halides, methoxy or ethoxy, more preferably Cl; in the presence of an organic base, preferably tertiary amine, more preferably triethylamine, diisopropylethylamine or N,N-diisopropylethylamine; a mixture of water and water-miscible polar or non-polar solvent to form
a reactive anhydride of the general formula III 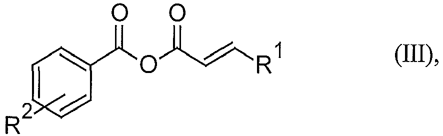 wherein
R¹ and R² are as defined above
that reacts with hyaluronic acid or a salt thereof in the presence of an organic base, preferably tertiary amine, more preferably triethylamine, diisopropylethylamine or N,N-diisopropylethylamine; a mixture of water and water-miscible polar or non-polar solvent, to form the photocurable ester derivative of hyaluronic acid or its salt of the general formula IV as defined in Claim 1 or Claim 2 that is dissolved in a carrying polymer of molecular weight from 5 x 10⁴ g/mol to 1 x 10⁶ g/mol, preferably 4 x 10⁵ g/mol in water to form a spinning solution that is spun by electrospinning.

9. The method according to Claim 8 **characterized in that** the α,β-unsaturated substituted acrylic acid is 5-membered or 6-membered aromatic or heteroaromatic acrylic acid of general formulae Ia or Ib 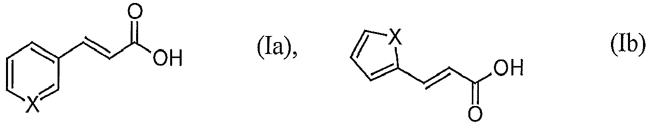 wherein X is selected from C, O, S, N, preferably O or N and the substituted benzoyl chloride of the general formula II is trichlorobenzoyl chloride, preferably α,β-unsaturated substituted acrylic acid is 3-(2-furyl)acrylic acid..

10. The method according to Claims 8 or 9 **characterized in that** the carying polymer is selected from a group comprising polyvinyl alcohol, polyacrylic acid, polyethylene oxide, polyvinyl pyrrolidone.

11. The method according to Claim 8 or 9 **characterized in that** the activation of the α,β-unsaturated substituted acrylic acid of the general formula I is carried out for 5 to 120 minutes at a temperature in a range from 0 °C to 60 °C, preferably 25 °C to 60 °C, more preferably for 30 minutes at 25°C.

12. The method according to Claim 8 **characterized in that** the amount of reactive anhydride mixed with hyaluronic acid or its salt corresponds to 0.01 to 5 equivalents, preferably 0.5 to 2 equivalents, more preferably 0,5 equivalents per hyaluronic acid dimer or its salt.

13. The method according to any one of Claims 8 to 12 **characterized in that** hyaluronic acid or its salt has a molecular weight from 5x10³ to 1.6x10⁶ g/mol, preferably from 1.5x10⁴ to 2.5x10⁵ g/mol, more preferably 8.5x10⁴ to 1.20x10⁵ g/mol.

14. The method according to any one of Claims 8 to 13 **characterized in that** forming of the photocurable ester derivative of hyaluronic acid or its salt is carried out for 1 to 48 hours, preferably 1 to 3 hours; at temperatures within the range of 0 °C to 80°C, preferably from 20 °C to 60 °C, most preferably at 25°C..

15. The method according to any one of Claims 8 to 14 **characterized in that** the water miscible polar or non-polar solvent is selected from a group comprising isopropanol, acetone, ethyl acetate, dimethyl sulfoxide, acetonitrile, dimethylformamide, tetrahydrofurane, preferably isopropanol, tetrahydrofurane, most preferably isopropanol; the amount of water in the mixture water and water miscible polar solvent being from 10 % to 99 %v/v, preferably 50 %v/v.

16. The method according to any one of Claims 8 to 15 **characterized in that** the concentration of the photocurable ester derivative of hyaluronic acid or its salt in the spinning solution is in the range of 0.5 to 12% (w/v), preferably 5 to 7.5% (w/v), more preferably 5% (w/v).

17. A method of photo-curing of nanofibers defined in any one of Claims 1 to 7 **characterized in that** the nanofibers are photocured by light irradiating within the range of UV-Vis wavelengths whereby cycloaddition of at least two ester groups of at least two molecules of hyaluronic acid or its salt of the formula IV occurs, forming the compound having cyclobutane ring of the general formula V 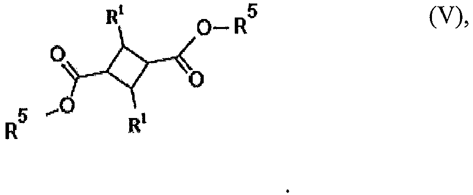 wherein
R¹ is as defined in Claim 1 and
R⁵ is a backbone chain of hyaluronic acid or its salt.

18. The method according to Claim 17 **characterized in that** UV-light has the wavelength range from 280 nm to 750 nm, preferably 302 nm; photocuring is being carried out for 2 min to 60 min, preferably for 3 min to 10 min..

19. Photocured nanofibers based on hyaluronic acid derivatives **characterized in that** they comprise the compound having cyclobutane ring of the formula V, defined in Claim 17.

20. A preparation comprising photocured nanofibers **characterized in that** the photocured nanofibers are as defined in Claim 19 and that the preparation is in a form selected from a group comprising layer, film, mat, wound dressing or tissue scaffold.

21. The preparation defined in Claim 20 for use in cosmetics, medicine or regenerative medicine.

22. The preparation defined in Claim 21 in wound care device, or in patches for external or internal use.

## Patentansprüche

1. Nanofasern auf der Basis von Esterderivate der Hyaluronsäure und deren Salzform, **dadurch gekennzeichnet, dass** sie ein lichtaushärtendes Esterderivat der Hyaluronsäure oder deren Salzform nach der allgemeinen Formel IV, 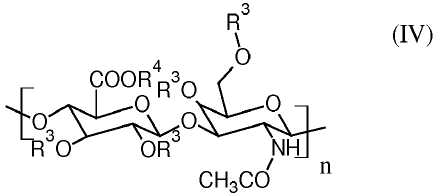
wobei n eine im Bereich von 1 bis 5000 Dimeren liegende ganze Zahl ist,
R³ unabhängig voneinander H oder COCHCHR¹ sind, wobei R¹ ein Aryl mit 5 oder 6 Gliedern oder ein Heteroaryl mit 5 oder 6 Gliedern ist, das mindestens ein oder mehrere identische oder unterschiedliche Heteroatome aufweist, die aus der aus der Gruppe ausgewählt sind, die N, O, S umfasst, sofern mindestens ein R³ des Derivates von COCHCHR¹ gebildet ist,
R⁴ entweder H⁺ oder ein pharmazeutisch akzeptierbares Salz ist;
und ein Trägerpolymer umfassen.

2. Nanofasern nach Anspruch 1, **dadurch gekennzeichnet, dass** R¹ aus der Gruppe ausgewählt ist, die Phenyl, Furyl, Furfuryl, Thienyl, Thiophenyl, Pyridil oder Imidazoyl umfasst;
R⁴ aus der Gruppe ausgewählt ist, die beliebige Ionen alkalischer Metalle oder Ionen alkalischer Erdmetalle, vorzugsweise Na⁺, K⁺, Mg²⁺ oder Li⁺, umfasst.

3. Nanofasern nach einem der Ansprüche 1 oder 2, **dadurch gekennzeichnet, dass** der Mengenanteil von COCHCHR¹ in dem lichtaushärtenden Esterderivat der Hyaluronsäure, bezogen auf 100 Dimere der Hyaluronsäure oder deren Salzform, im Bereich von 0,1 bis 20 %, vorzugsweise von 5 bis 10 % liegt und vorzüglicherweise 5 % beträgt.

4. Nanofasern nach einem der Ansprüche 1 bis 3 **dadurch gekennzeichnet, dass** das Trägerpolymer aus der Gruppe ausgewählt ist, die Polyvinylalkohol, Polyacrylsäure, Polyethylenoxid und Polyvinylpyrrolidon umfasst, wobei der Mengenanteil des Trägerpolymers im Bereich von 50 bis 99 Gew.-%, vorzugsweise von 70 bis 90 Gew-% liegt und vorzüglicherweise 80 Gew.-% beträgt.

5. Nanofasern nach einem der Ansprüche 1 bis 4, **dadurch gekennzeichnet, dass** sie zusätzlich ein biologisch verträgliches Polymer, vorzugsweise Carboxymethylcellulose, Gelatine, Chitosan, Polycaprolacton, Polymilchsäure, Polyamid, Polyurethan, Poly-(milch/glykol)-säure, sowie deren Gemische oder deren Kopolymere umfassen.

6. Nanofasern nach einem der Ansprüche 1 bis 5, **dadurch gekennzeichnet, dass** ihr durchschnittlicher Durchmesser im Bereich von 1 bis 500 nm, vorzugsweise von 99 bis 300 nm, liegt.

7. Nanofasern nach einem der Ansprüche 1 bis 6, **dadurch gekennzeichnet, dass** sie in der Gelform vorkommen.

8. Verfahren zur Synthese von Nanofasern nach einem der Ansprüche 1 bis 7, **dadurch gekennzeichnet, dass** α,β-ungesättigte substituierte Acrylsäure nach der allgemeinen Formel I, 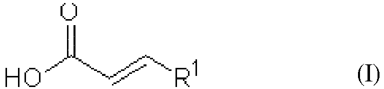 wobei
R¹ ein Aryl mit 5 oder 6 Gliedern oder ein Heteroaryl mit 5 oder 6 Gliedern ist, das mindestens ein oder mehrere identische oder unterschiedliche Heteroatome aufweist, die aus der Gruppe ausgewählt sind, die N, O oder S umfasst,
mit einem substituierten oder nicht substituierten Benzoylchlorid oder mit dessen Derivaten nach der allgemeinen Formel II aktiviert ist, 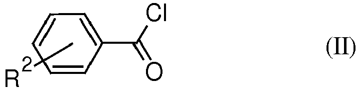 wobei R² von einem oder mehreren Substituenten gebildet ist, die aus der Gruppe ausgewählt sind, die H, -NO₂, -COOH, Halide, C₁-C₆-Alkylkoxy-, vorzugsweise Halide, Methoxy- oder Ethoxy, vorzüglicherweise Cl, umfasst;
und zwar in Gegenwart von einer organischen Basis, vorzugsweise einem tertiären Amin, vorzüglicherweise Triethylamin, Diisopropyl-Ethylamin oder N-N-Diisopropyl-Ethylamin; von einem Gemisch, das aus Wasser und einem wassermischbaren polaren oder unpolaren Lösungsmittel besteht, unter Bildung von einem reaktiven Anhydrit nach der allgemeinen Formel III, 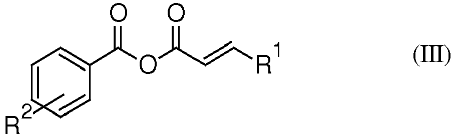 wobei
R¹ und R² den obigen Definitionen entsprechen,
der mit der Hyaluronsäure oder deren Salzform in Gegenwart von einer organischen Basis, vorzugsweise einem tertiären Amin, vorzüglicherweise Triethylamin, Diisopropyl-Ethylamin oder N-N-Diisopropyl-Ethylamin; von einem Gemisch von Wasser und einem wassermischbaren polaren oder unpolaren Lösungsmittel, reagiert, unter Bildung des lichtaushärtenden Esterderivats der Hyaluronsäure oder deren Salzform nach der allgemeinen Formel IV, welches Derivat im Anspruch 1 oder im Anspruch Claim 2 definiert ist, und welches in einem Trägerpolymer mit dem Molekulargewicht im Bereich von 5 x 10⁴ g/mol bis 1 x 10⁶ g/mol, vorzugsweise mit dem Molekulargewicht von 4 x 10⁵ g/mol in Wasser aufgelöst ist, unter Bildung einer zu verspinnenden Lösung, die in einem Electrospinning-Verfahren versponnen wird.

9. Verfahren nach Anspruch 8, **dadurch gekennzeichnet, dass** die α,β-ungesättigte substituierte Acrylsäure eine aromatische oder heteroaromatische Acrylsäure mit 5 oder 6 Gliedern nach einer der allgemeinen Formeln Ia oder Ib ist, 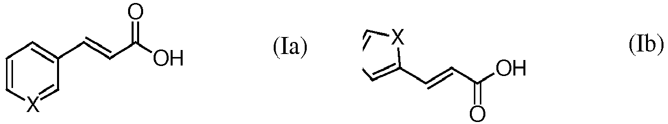 wobei X aus der Gruppe ausgewählt ist, die C, O, S, N, vorzugsweise O oder N umfasst, und das substituierte Benzoylchlorid nach der allgemeinen Formel II Trichlorobenzoylchlorid ist, wobei die α,β-ungesättigte substituierte Acrylsäure vorzugsweise die 3-(2-Furyl)Acrylsäure ist.

10. Verfahren nach einem der Ansprüche 8 oder 9, **dadurch gekennzeichnet, dass** das Trägerpolymer aus der Gruppe ausgewählt ist, die Polyvinylalkohol, Polyacrylsäure, Polyethylenoxid und Polyvinylpyrrolidon umfasst.

11. Verfahren nach einem der Ansprüche 8 oder 9, **dadurch gekennzeichnet, dass** die Aktivierung der α,β-ungesättigten substituierten Acrylsäure nach der allgemeinen Formel I während des Zeitraums von 5 bis 120 Minuten und bei einer Temperatur im Bereich von 0 °C bis 60 °C, vorzugsweise von 25 °C bis 60 °C, vorzüglicherweise 30 Minuten bei der Temperatur von 25 °C verläuft.

12. Verfahren nach Anspruch 8, **dadurch gekennzeichnet, dass** die Mengen des mit der Hyaluronsäure oder deren Salzform vermischten reaktiven Anhydrit einem Anteil entspricht, der 0,01 bis 5 Äquivalente, vorzugsweise 0,5 bis 2 Äquivalente, vorzüglicherweise ein 0,5 Äquivalent per Dimer der Hyaluronsäure und deren Salzform, beträgt.

13. Verfahren nach einem der Ansprüche 8 bis 12, **dadurch gekennzeichnet, dass** die Hyaluronsäure oder deren Salzform ein Molekulargewicht im Bereich von 5x10³ bis 1,6x10⁶ g/mol, vorzugsweise von 1,5x10⁴ bis 2,5x10⁵ g/mol, vorzüglicherweise von 8,5x10⁴ bis 1,20x10⁵ g/mol aufweist.

14. Verfahren nach einem der Ansprüche 8 bis 13, **dadurch gekennzeichnet, dass** die Bildung des lichtaushärtenden Esterderivats der Hyaluronsäure oder deren Salzform im Zeitraum von 1 bis 48 Stunden, vorzugsweise von 1 bis 3 Stunden, und bei einer Temperatur im Bereich von 0 °C bis 80°C, vorzugsweise von im Bereich 20 °C bis 60 °C, vorzüglicherweise von 25°C, verläuft.

15. Verfahren nach einem der Ansprüche 8 bis 14, **dadurch gekennzeichnet, dass** das wassermischbare polare oder unpolare Lösungsmittel aus der Gruppe ausgewählt ist, die Isopropanol, Aceton, Ethylacetat, Dimethylsulfoxid, Acetonitril, Dimethylformamid und Tetrahydrofuran, vorzugsweise Isopropanol und Tetrahydrofuran, vorzüglicherweise lediglich Isopropanol umfasst, wobei der Mengenanteil von Wasser in der Mischung von Wasser und dem wassermischbaren polaren Lösungsmittel im Bereich von 10 Vol.-% bis 99 Vol.-% liegt und vorzugsweise 50 Vol.% beträgt.

16. Verfahren nach einem der Ansprüche 8 bis 15, **dadurch gekennzeichnet, dass** die Konzentration des lichtaushärtenden Esterderivats der Hyaluronsäure oder deren Salzform in der zu verspinnenden Lösung im Bereich von 0,5 bis 12 Gew./Vol.-%, vorzugsweise im Bereich von 5 bis 7,5 Gew./Vol.-% liegt und vorzüglicherweise 5 Gew./Vol.-% beträgt.

17. Verfahren zur Lichtaushärtung von Nanofasern nach einem der Ansprüche 1 bis 7, **dadurch gekennzeichnet, dass** die Nanofasern lichtausgehärtet werden, indem sie der Bestrahlung mit Licht unterzogen werden, dessen Wellenlänge in dem UV-VIS Band liegt, wobei Zykloaddition von wenigstens zwei Estergruppen mit wenigstens zwei Molekülen der Hyaluronsäure oder deren Salzform nach der allgemeinen Formel IV erfolgt, wodurch eine Verbindung gebildet wird, die einen Zyklobutan-Zyklus nach der allgemeinen Formel V umfasst, 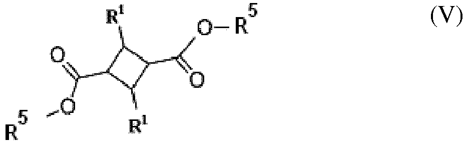 wobei
R¹ der im Anspruch 1 angeführten Definition entspricht und
R⁵ eine Rückgratkette der Hyaluronsäure oder deren Salzform ist.

18. Verfahren nach Anspruch 17, **dadurch gekennzeichnet, dass** die Wellenlänge des UV-Lichts im Bereich von 280 nm bis 750 nm liegt und vorzugsweise 302 nm beträgt, wobei die Lichtaushärtung 2 bis 60 Minuten, vorzugsweise 3 bis 10 Minuten, lang durchgeführt wird.

19. Lichtausgehärtete Nanofasern auf der Basis der Derivate der Hyaluronsäure, **dadurch gekennzeichnet, dass** sie die Verbindung mit dem Zyklobutan-Zyklus nach der allgemeinen Formel V umfassen, die im Anspruch 17 definiert ist.

20. Ein Präparat, das lichtausgehärtete Nanofasern umfasst, **dadurch gekennzeichnet, dass** die lichtausgehärteten Nanofasern im Anspruch 19 definiert sind und dass das Präparat in einer Form vorliegt, die aus der Gruppe ausgewählt ist, die eine Schicht, einen Film, eine Matte, einen Wundenverband oder ein Gewebegerüst umfasst.

21. Das Präparat nach Anspruch 20, zur Anwendung auf den Gebieten der Kosmetik, allgemeinen Medizin oder regenerativen Medizin.

22. Das Präparat nach Anspruch 21, zur Anwendung als ein Bestandteil von Wundheilungssystemen oder in Pflastern, die zum äußeren oder inneren Gebrauch bestimmt sind.

## Revendications

1. Nanofibres à base de dérivés ester de l'acide hyaluronique et de son sel, **caractérisées en ce qu'**ils comprennent un dérivé ester photodurcissable de l'acide hyaluronique ou son sel selon la formule générale IV 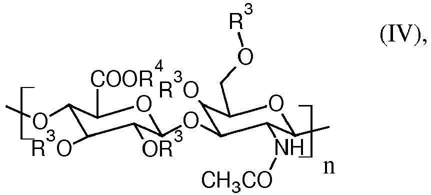
dans laquelle n est un nombre entier dans la plage de 1 à 5000 dimères,
R³ représentent indépendamment H ou COCHCHR¹ où R¹ représente un groupe aryle à 5 ou 6 chaînons ou un groupe hétéroaryle à 5 ou 6 chaînons ayant au moins un ou plusieurs hétéroatomes identiques ou différents choisis parmi le groupe comprenant N, O, S, pourvu qu'au moins un R³ du dérivé soit COCHCHR¹;
R⁴ représente H⁺ ou un sel pharmaceutiquement acceptable;
et au moins un polymère porteur.

2. Nanofibres selon la revendication 1, **caractérisées en ce que** R¹ est choisi parmi le groupe comprenant phényle, furyle, furfuryle, thiényle, thiofényle, pyridyle ou imidazoyle; R⁴ est choisi parmi un groupe comprenant quelconque des ions de métaux alcalins ou des ions de métaux alcalino-terreux, de préférence Na⁺, K⁺, Mg² + ou Li⁺.

3. Nanofibres selon l'une quelconque des revendications 1 à 2, **caractérisées en ce que** la quantité de COCHCHR¹ dans le dérivé ester de l'HA photodurcissable est de 0,1 à 20 % par rapport à 100 dimères de l'acide hyaluronique ou de son sel, de préférence de 5 à 10 %, plus préférablement 5 %.

4. Nanofibres selon l'une quelconque des revendications 1 à 3, **caractérisées en ce que** le polymère porteur est choisi parmi le groupe comprenant l'alcool polyvinylique, l'acide polyacrylique, l'oxyde de polyéthylène, la polyvinylpyrrolidone; la quantité du polymère porteur étant dans la plage de 50 % (poids / poids) à 99 % (poids / poids), de préférence dans la plage de 70 à 90 % (poids / poids), plus préférablement 80 % (poids / poids).

5. Nanofibres selon l'une quelconque des revendications 1 à 4, **caractérisées en ce qu'**elles comprennent en outre un polymère biologiquement compatible, de préférence la carboxyméthylcellulose, la gélatine, le chitosane, la polycaprolactone, l'acide polylactique, le polyamide, le polyuréthane, l'acide poly (lactide-co-glycolique); et leur mélange ou leurs copolymères.

6. Nanofibres selon l'une quelconque des revendications 1 à 5, **caractérisées en ce que** leur diamètre moyen est compris entre 1 et 500 nm, de préférence 99 à 300 nm.

7. Nanofibres selon l'une quelconque des revendications 1 à 6, **caractérisées en ce qu'**elles sont sous forme de gel.

8. Procédé de synthèse de nanofibres selon l'une quelconque des revendications 1 à 7, **caractérisé en ce que** l'acide acrylique α,β-insaturé substitué de la formule générale I 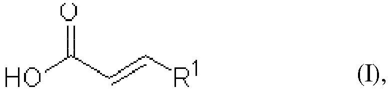 où
R¹ est un aryle à 5 ou 6 chaînons ou un hétéroaryle à 5 ou 6 chaînons ayant au moins un ou plusieurs hétéroatomes identiques ou différents choisis dans le groupe comprenant N, O ou S; est activé avec le chlorure de benzoyle substitué ou non substitué ou ses dérivés de la formule générale II 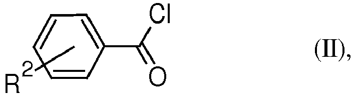 dans laquelle R² représente un ou plusieurs substituants choisis parmi le groupe comprenant H, -NO₂, -COOH, des halogénures, des C₁-C₆-alkylcoxy, de préférence des halogénures, méthoxy ou éthoxy, plus préférablement Cl; en présence d'une base organique, de préférence de l'aminé tertiaire, plus préférablement de la triéthylamine, de la diisopropyléthylamine ou de la N, N-diisopropyléthylamine; un mélange d'eau et de solvant polaire ou non polaire miscible avec de l'eau pour former un anhydride réactif de la formule générale III 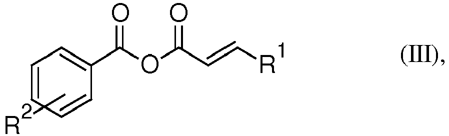 où
R¹ et R² sont tels que définis ci-dessus
et qui réagit avec l'acide hyaluronique ou un sel de celui-ci en présence d'une base organique, de préférence de l'amine tertiaire, plus préférablement de la triéthylamine, de la diisopropyléthylamine ou de la N, N-diisopropyléthylamine; un mélange d'eau et de solvant polaire ou non polaire miscible avec de l'eau,
pour former le dérivé ester photodurcissable de l'acide hyaluronique ou son sel de la formule générale IV définie dans la revendication 1 ou la revendication 2 qui est dissous dans un polymère porteur de poids moléculaire de 5 x 10⁴ g / mol à 1 x 10⁶ g / mol, de préférence 4 x 10⁵ g / mol dans l'eau pour former une solution de filage qui est filée par électrospinning.

9. Procédé selon la revendication 8, **caractérisé en ce que** l'acide acrylique α,β-insaturé substitué est l'acide acrylique aromatique ou hétéroaromatique à 5 chaînons ou à 6 chaînons selon les formules générales Ia ou Ib, 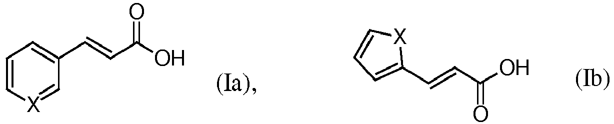 dans lequel X est choisi parmi C, O, S, N, de préférence O ou N, et le chlorure de benzoyle substitué de formule générale II est le chlorure de trichlorobenzoyle, de préférence l'acide acrylique α,β-insaturé substitué est l'acide 3- (2-furyle) acrylique.

10. Procédé selon les revendications 8 ou 9, **caractérisé en ce que** le polymère porteur est choisi parmi un groupe comprenant l'alcool polyvinylique, l'acide polyacrylique, l'oxyde de polyéthylène, la polyvinylpyrrolidone.

11. Procédé selon la revendication 8 ou 9, **caractérisé en ce que** l'activation de l'acide acrylique α,β-insaturé substitué de la formule générale I est effectuée pendant 5 à 120 minutes à une température dans la plage de 0 °C à 60 °C, de préférence de 25 °C à 60 °C, plus préférablement pendant 30 minutes à 25 °C.

12. Procédé selon la revendication 8, **caractérisé en ce que** la quantité d'anhydride réactif mélangé avec de l'acide hyaluronique ou son sel correspond à 0,01 à 5 équivalents, de préférence à 0,5 à 2 équivalents, plus préférablement 0,5 équivalent par un dimère de l'acide hyaluronique ou son sel.

13. Procédé selon l'une quelconque des revendications 8 à 12, **caractérisé en ce que** l'acide hyaluronique ou son sel a le poids moléculaire de 5x10³ à 1,6x10⁶ g / mole, de préférence de 1,5x10⁴ à 2,5x10⁵ g / mol, plus préférablement de 8,5x10⁴ à 1,20x10⁵ g / mol.

14. Procédé selon l'une quelconque des revendications 8 à 13, **caractérisé en ce que** la formation du dérivé ester photodurcissable de l'acide hyaluronique ou de son sel est effectuée pendant 1 à 48 heures, de préférence 1 à 3 heures; aux températures dans la plage de 0 °C à 80 °C, de préférence de 20 °C à 60 °C, plus préférablement à 25 °C.

15. Procédé selon l'une quelconque des revendications 8 à 14, **caractérisé en ce que** le solvant polaire ou non polaire miscible avec de l'eau est choisi parmi le groupe comprenant l'isopropanol, l'acétone, l'acétate d'éthyle, le diméthylsulfoxyde, l'acétonitrile, le diméthylformamide, le tétrahydrofurane, de préférence l'isopropanol, le tétrahydrofurane, le plus préférablement l'isopropanol; la quantité de l'eau dans le mélange de l'eau et du solvant polaire miscible avec de l'eau étant de 10 % à 99 % vol / vol, de préférence 50 % vol / vol.

16. Procédé selon l'une quelconque des revendications 8 à 15, **caractérisé en ce que** la concentration du dérivé ester photodurcissable de l'acide hyaluronique ou de son sel dans la solution de filage est comprise entre 0,5 et 12 % (poids / vol), de préférence 5 à 7,5% (poids / vol), plus préférablement à 5% (poids / vol).

17. Procédé de photo-réticulation des nanofibres définies dans l'une quelconque des revendications 1 à 7, **caractérisé en ce que** les nanofibres sont réticulées par irradiation lumineuse dans la gamme des longueurs d'onde UV-Vis, grâce à quoi la cycloaddition d'au moins deux groupes ester d'au moins deux molécules de l'acide hyaluronique ou de son sel de la formule IV se produit, formant le composé ayant un cycle de cyclobutane de la formule générale V 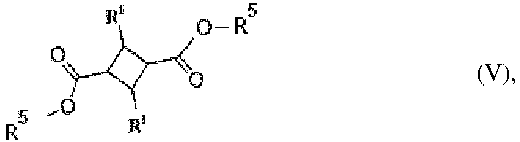 où
R¹ est tel que défini dans la revendication 1 et R^{S} est une chaîne de squelette de l'acide hyaluronique ou son sel.

18. Procédé selon la revendication 17, **caractérisé en ce que** la lumière UV a la plage de longueur d'onde de 280 nm à 750 nm, de préférence 302 nm; la réticulation est effectuée pendant 2 min à 60 min, de préférence pendant 3 min à 10 min.

19. Nanofibres photodurcies à base de dérivés de l'acide hyaluronique, **caractérisées en ce qu'**elles comprennent le composé ayant un cycle de cyclobutane de la formule V, défini dans la revendication 17.

20. Une préparation comprenant des nanofibres photodurcies, **caractérisée en ce que** les nanofibres photodurcies sont telles que définies dans la revendication 19 et que la préparation est sous la forme choisie parmi le groupe comprenant une couche, un film, un galette, un pansement ou un squelette de tissu.

21. La préparation définie dans la revendication 20 pour l'utilisation dans les cosmétiques, la médecine ou la médecine régénératrice.

22. Préparation définie dans la revendication 21 dans un moyen de soins des plaies, ou dans les emplâtres pour usage externe ou interne.
